(19) Europäisches Patentamt
European Patent Office
Office européen des brevets

(11) **EP 3 425 404 A1**

(12) **EUROPEAN PATENT APPLICATION**
published in accordance with Art. 153(4) EPC

(43) Date of publication:
**09.01.2019 Bulletin 2019/02**

(51) Int Cl.:
**G01N 33/68** (2006.01)     **G01N 27/447** (2006.01)
**C12Q 1/42** (2006.01)

(21) Application number: **17759893.5**

(22) Date of filing: **27.02.2017**

(86) International application number:
**PCT/JP2017/007406**

(87) International publication number:
**WO 2017/150427 (08.09.2017 Gazette 2017/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **29.02.2016 JP 2016036969**

(71) Applicants:
• **KOBAYASHI, Tsuneo**
  **Chiba-shi, Chiba 261-0004 (JP)**

• **Uchino, Yuriko**
  **Chiba-shi, Chiba 261-0004 (JP)**

(72) Inventor: **KOBAYASHI, Tsuneo**
  **Chiba-shi**
  **Chiba 261-0004 (JP)**

(74) Representative: **DREISS Patentanwälte PartG
  mbB**
  **Friedrichstraße 6**
  **70174 Stuttgart (DE)**

(54) **DATA COLLECTION METHOD TO BE USED WHEN CLASSIFYING CANCER LIFE**

(57)     Blood serum is applied on a support that has been impregnated in a buffer solution, the support is fractionated by electrophoresis at a predetermined liquid temperature to isolate proteins in the blood serum, an ALP isozyme is detected by color-developing with an ALP isozyme staining solution, the mobility, chromosome shape, density, and the like of each isozyme are determined by matching the protein fraction image against the ALP isozyme, development of a minute cancer that is occurring is discovered, and also the risk of tumor and risk of cancer are evaluated by matching against the analysis results of a tumor marker to classify the life of the cancer for early cancer discovery.

FIG. 2

EP 3 425 404 A1

**Description**

Technical Field

The present disclosure relates to a method for collecting data to be used for biochemical detection of minute cancer by examining blood or urine of a cancer suspect and early discovery evaluation of cancer in a quantitative manner, in particular, a method for collecting data to be used for classifying the life of cancer by detecting both the minute cancer at a preclinical cancer stage and a clinical cancer stage in the life of cancer and performing accurate data analysis of the risk of cancer.

Background Art

For cancer therapy, early discovery and diagnosis of cancer is believed to be important. In particular, if it is minute cancer found at early stage, there is even a case in which the cancer is fully cured by natural healing power. Accordingly, numerous methods for early discovery of cancer including stomach cancer, lung cancer, colon cancer, liver cancer, and breast cancer have been suggested and they are also actually carried out.

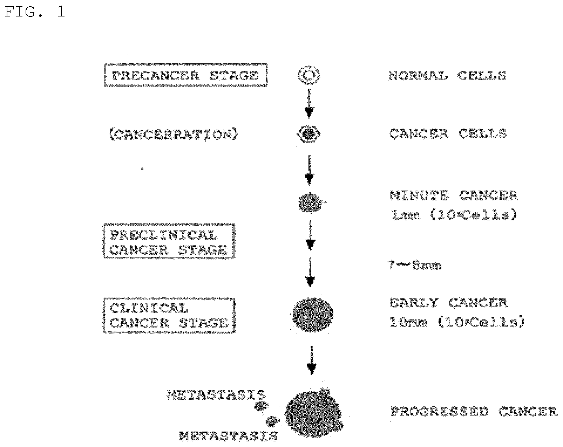

Furthermore, as a result of repetitive division, cancerized cells produce cancer tissues and are shown as a disease . Life of cancer is classified into 3 stages, i.e., "precancer stage (normal stage) ", "preclinical cancer stage", and "clinical cancer stage as shown in Fig. 1. Although the canceration of cells occurs during a precancer stage, the colony of cancer cells before preclinical cancer stage remains minute, and thus it is difficult to determine visually the existence of cancer at that time. In general, visually determinable cancer is generally cancer having diameter of 10 mm or more at a clinical cancer stage, which consists of about one billion ($10^9$) cancer cells or more. Those recognized generally as a disorder and regarded as a subject for treatment indicate the cancer at a clinical cancer stage.

Cancer cells often produce special components that are absent in normal cells. Furthermore, there is also a case in which a component present in an extremely small amount in normal human being is produced in a large amount in a patient having cancer. Those components specific to cancer cells are referred to as a "tumor marker", in the same meaning as an indicator (i.e., marker) of cancer cells.

As for the tumor marker, there are various markers such as "alpha-feto protein, $\alpha$-fetoprotein, AFP" relating to liver cancer and "carcinoembryonic antigen, CEA" relating to stomach cancer or colon cancer, which are the proteins produced only in cancer cells or an embryo, tumor-specific antigen which reacts with "sugar chain antigen 19-9, carbohydrate antigen 19-9, CA19-9" relating to pancreatic cancer, and hormones including "gonadotropin, human chorionic gonadotropin, HCG" relating to uterine choriocarcinoma, "calcitonin" relating to parathyroid cancer and cancerous "alkaline phosphatase (hereinbelow, described as "ALP") relating to bone cancer or liver cancer, and the like.

Under the right conditions, the ALP is an important tumor marker and an enzyme having an activity of dissociating phosphoric acid, and, in human body, several types with different molecular weight are found in each organ such as placenta and small intestine, in addition to the above-mentioned bone and liver. Like the ALP, when plural kinds of enzymes having the same activity are present in each organ, they are called "isozymes". For example, in a patient suffering from bone cancer, liver cancer, multiple-myelomatosis and like, isozymes having molecular weight different from the enzymes of a normal person are produced. In those cancer cells, the normal gene activity of regulating each isozyme is lost so that the isozyme undergoes a different pattern change. Accordingly, if those isozyme patterns are determined and evaluated appropriately, minute cancer occurring in bone, liver, colon, lung, or the like can be discovered.

As such, a method of determining the ALP isozyme by electrophoresis has been suggested in related art. The detection method is a method for detecting the isozyme in which blood serum proteins are isolated by electrophoresis and then reacted with a naphthol-based phosphoric acid compound, and the released naphthol-based materials are subjected to diazotization using diazonium salt to perform color development.

This method for determining the ALP isozyme is carried out by following means, for example.

(1) Sample application

First, cellogel immersed in advance for more than an hour in a buffer solution for electrophoresis is cut in half to a size of 2.5 × 6 cm. On a spot at a distance of 1 cm from the negative electrode side thereof, 8 to 10 $\mu$l of blood serum separated from blood of a person as a cancer evaluation subject is applied. In case of high activity, the application amount can be adjusted.

(2) Electrophoresis

Electrophoresis is carried out at 100 V until albumin migrates to 1 cm from the positive electrode terminal. It is preferable to perform cooling at that moment.

(3) After completion of staining electrophoresis

After completing the staining electrophoresis, the cellogel is further cut in half. One piece is subjected to protein staining while the other piece is subjected to ALP staining. As for the ALP staining, the electrophoresed membrane is overlaid on top of a filter paper smeared with staining solution such that no air is introduced between the paper and membrane, and the resultant is heated for 1 hour or so at 37°C in dark conditions. After the staining, fixing is carried out according to immersion in 1% acetic acid.

(4) Method for evaluation

Protein fraction image and the ALP isozymes are matched to each other, and mobility, chromosome shape, density and the like of each isozyme are examined. Although I to VI bands are detected from blood serum by electrophoresis, when classification is made in terms of the antigenicity, they belong to five types of hepatic ALP, placental ALP, small intestinal ALP, systemic ALP, and bone type ALP, which are different in originating organs, and it is considered that those five types of isozymes are different in terms of the modification form or sugar chain.

Blood serum ALP of a normal human being mainly consists of hepatic type ($\alpha_2$ region), and those with $\alpha_2 > \alpha_2\beta$ are present in a large amount. From the secretion type with blood type O and B, the small intestinal ALP is detected at high frequency. In childhood period, the bone type is shown (i.e., stained as an active band with broad width), and, in late pregnancy period, heat-resistant placental ALP is shown. The ALP showing an increase in liver or bile duct disorder is mainly in $\alpha_2$ region originating from liver. However, in cholestasis either inside or outside a liver, metastatic liver cancer, drug-related disorder, or the like, the high-molecular-weight isozyme in $\alpha_1$ region is shown. In chronic hepatitis and liver cirrhosis, the small-intestinal ALP is shown at high frequency. The bone type ALP is increased in rickets, Paget's disease, hyperparathyroidism, hyperthyroidism, bone fracture, bone metastasis of cancer, osteosarcoma, or the like. In a patient having artificial dialysis, there is a case in which the ALP of bone type, small-intestinal type, or the like is shown. As for the abnormality of the ALP detected by electrophoresis, there is an abnormality caused by tumor generation, binding with immunoglobulin, and modification with sialic acid or neuraminidase.

(5) ALP isozyme resulting from tumor generation

As for the ALP resulting from tumor generation, which is detected by the aforementioned evaluation method, there are placental Regan ALP and Nagao ALP, and also small-intestinal Kasahara ALP. The placental ALP is detected as a sharp band. The placental ALP is detected at high frequency from ovarian cancer or uterine cancer, and it is detected from a cancer patient at a frequency of from 10 to 15%. According to cellulose acetate membrane electrophoresis, the small-intestinal Kasahara ALP is electrophoresed at the position of the ALP 1. However, by a treatment with Triton X-100, the mobility of the ALP 1 can be shifted to a negative electrode side, and thus the evaluation can be achieved. In case of 5% polyacrylamide gel electrophoresis, it can be determined as it is electrophoresed at the most positive electrode side. It is furthermore characterized by being inhibited by L-phenylalanine. Kasahara ALP is detected from about 15% of cancer patients.

In blood serum of a human being who is either healthy or has various disorders, various kinds of the ALP isozymes including hepatic, bone type, placental, and small-intestinal isozymes are present as shown in Table 4. As there is a significant difference among them in terms of the electrophoresis mobility, heat resistance, degree of inhibition by various amino acids, cross-reactive immune response, or the like, it is used for discriminating one type from another.

[Table 4]

| Properties of various isozymes of blood serum alkaline phosphatasete | | | | | | |
|---|---|---|---|---|---|---|
| | Hepatic cancer type | Hepatic type (high molecular weight) | Hepatic type | Bone type | Placenta type (ALP) | Small-intestine type |
| Electrophoresis range | $\alpha_1 \sim \alpha_2$ | $(\alpha_1)$ | $\alpha_2$ | $\alpha_2\beta$ | $\alpha_2\beta$ | $\beta$ |
| Heat resistance (Deactivation rate %) 56°C 5 minutes 65°C 10 minutes | 5 90 | 30 100 | 60 100 | 80 100 | 0 0 | 70 100 |
| Inhibitor (Inhibition degree %) | | | | | | |

(continued)

| Properties of various isozymes of blood serum alkaline phosphatasete | | | | | | |
|---|---|---|---|---|---|---|
| | Hepatic cancer type | Hepatic type (high molecular weight) | Hepatic type | Bone type | Placenta type (ALP) | Small-intestine type |
| Phenylalanine | 80 | 10 | 10 | 10 | 70 | 60 |
| Leucine | 10 | 0 | 0 | 0 | 5 | 10 |
| Homoargine | 18 | 60 | 60 | 60 | 15 | 20 |
| Neuraminidase susceptibility | + | + | + | + | + | - |
| Km (m mol/l) | 1.7 | 1.7 | 1.7 | 2.0 | 2.0 | 1.7 |
| Cross-reactive immune response | | | | | | |
| Anti-hepatic ALP | - | 3+ | 3+ | 3+ | - | - |
| Anti-placental ALP | + | - | - | - | 3+ | + |
| Anti-small-intestinal ALP | 3+ | - | - | - | + | 3+ |
| (Note) 1) There are immunoglobulin binding type ($\beta$ and $\gamma$) and high molecular weight small-intestinal type ($\alpha_2\beta$, lipid complex) other than the isozymes described above. 2) Mobility of the hepatic type (high molecular weight) varies depending on a support. Amino acid inhibition concentration is 5 mM for phenylalanine and homoarginine, and 0.4 mM for leucine. Km is based on Kind-King method with addition of $Mg^{2+}$. 3) Nagao type of tumor-generating APL has 90% phenylalanine inhibition and 50% leucine inhibition. | | | | | | |

The ALP is a dimer having molecular weight of 120,000 to 160,000. Being bound to a cellular membrane of each tissue cell, it is present as a complex. However, in case of having a damage in tissue, the large (high) molecular ALP composed of fine fragments of cellular membrane may be released into blood. According to general electrophoresis, small molecular ALP like the ALP with hepatic, bone type, placental, or small-intestinal origin migrates between $\alpha 1$ and $\beta$. However, mobility of a high molecular ALP varies depending on the type of a support (in particular, presence or absence of molecular sieve effect) . On a cellulose acetate membrane or agar, it is divided into a $\alpha_1$ region (originating from liver) and a component remaining on an original spot (the latter is lost during staining, and thus not detected). However, on a polyacrylamide gel or a starch gel, smaller mobility is yielded due to the molecular sieve effect, and thus it is divided into two fractions, i.e., the original spot region and a fraction which migrates just slightly therefrom.

On a polyacrylamide gel, the hepatic cancer type ALP to be described below is detected as a component having higher mobility than the hepatic type. However, on a cellulose acetate membrane or agar, it is overlapped with the hepatic type, thus not allowing any detection. Furthermore, the immunoglobulin binding type ALP is also well separated from the small-intestinal type on a polyacrylamide gel. As such, for an analysis of blood serum ALP isozymes based on electrophoresis, a cellulose acetate membrane or agar is used in combination with polyacrylamide gel.

Various kinds of techniques relating to a system for predicting, based on a result of cancer diagnosis, ever changing cancer onset risk under specific conditions are suggested. For example, like "Cancer onset risk prediction system and method, and cancer derivative method" of JP 2006-302222 A as Patent Literature 1, a prediction system provided at least with a calculating means and a memorizing means for predicting clinical cancer onset risk at specific conditions is suggested in which the memorizing means is provided with high-accuracy cancer medical examination clustering database, in which previous medical examination results of specific high-accuracy cancer medical examination allowing detection of preclinical cancer are classified, based on specific numbers representing the possibility of existence of cancer, into a normal state, a normal appearance state in which the possibility of existence of the cancer cannot be denied, and a clinical cancer state, and recorded such that data can be referenced for the each classification, and high-accuracy cancer medical re-examination result database in which, with regard to the high-accuracy cancer medical examination clustering database, results of high-accuracy cancer medical re-examination received for the each classification after specific time period are recorded such that the data can be referenced for the each classification, and the prediction system is provided with a clinical cancer state distribution calculating means for calculating correlation between the high-accuracy cancer medical examination clustering data and high-accuracy cancer medical re-examination result data and clinical cancer state distribution by having the result of the high-accuracy cancer medical examination or the specific time period as a variable, and a simulating means for yielding a prediction of the possibility that, after the specific time period calculated by the medical examination result, clinical cancer is developed in a specific examinee who has received with the high-accuracy cancer medical examination.

Citation List

Patent Literature

Patent Literature 1: JP 2006-302222 A

Summary of Invention

Technical Problem

However, according to the method for determining the ALP isozymes described above, it is difficult to detect cancer if the cancer does not grow to a certain size. As such, according to the conventional method for finding and evaluating cancer using the isozymes, each of the sensitivity and specificity is low. The sensitivity indicates a ratio of cancer patients who are clearly evaluated to have cancer while the specificity indicates a ratio of healthy people who are clearly evaluated to be free of any cancer. Accordingly, the conventional method for finding cancer by using the isozymes is performed as a kind of supplementary test during the processes of diagnosing cancer, or a test for observing a progress during a treatment of cancer.

Inventors of the present invention directed their attention to a method for biochemical detection of minute cancer by using, as an indicator of the existence of minute cancer, a pattern change at set time interval of the ALP isozyme and $\Delta$CEA (carcinoembryonic antigen) or $\Delta$ferritin. During the canceration process regarded as a de-differentiation or abnormal differentiation phenomenon, the ALP plays an important role. Within a range in which the total ALP activity in blood serum has a normal value, proliferation of cancer cells and patterns of the ALP I to the ALP IV isozymes have a close relationship. Thus, based on a change ($\Delta$) of a tumor marker during constant time period and analysis of the isozyme, it is possible to predict minute cancer with cell number of $10^4$ to $10^9$ (i.e., 10 $\mu$g to 1 g in weight) . Namely, invented is a new method for evaluating early cancer based on analysis of a pattern change of the ALP isozyme.

As for the tumor marker, there are various kinds such as "alpha-feto protein, $\alpha$-fetoprotein, AFP" relating to liver cancer and "carcinoembryonic antigen, CEA" relating to stomach cancer or colon cancer, which are the proteins produced in cancer cells, tumor-specific antigen which reacts with "sugar chain antigen 19-9, carbohydrate antigen 19-9, CA19-9" relating to pancreatic cancer, and hormones including "gonadotropin, human chorionic gonadotropin, HCG" relating to uterine choriocarcinoma, "calcitonin" relating to parathyroid cancer and cancerous "alkaline phosphatase (hereinbelow, described as "ALP") relating to bone cancer or liver cancer, and the like.

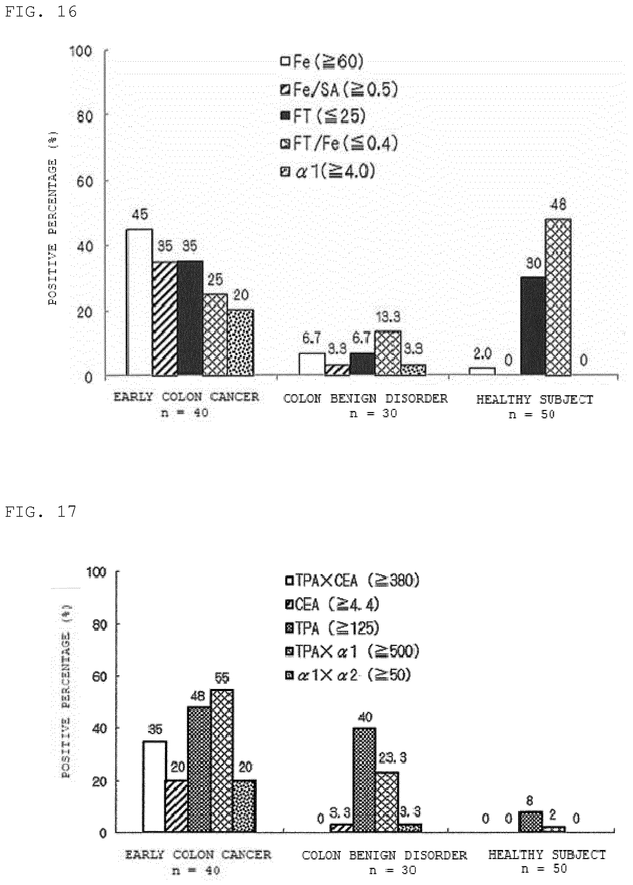

Fig. 16 is a graph illustrating the positive percentage of each tumor marker in people having early colon cancer, people having benign colon disorder, or healthy people. Fig. 17 is a graph illustrating the positive percentage of each tumor marker in people having early colon cancer, people having benign colon disorder, or healthy people.

By using a single tumor marker, minute cancer occurring in bone, liver, intestine, lung, or the like can be discovered. However, as pointed out by many oncologists before, for increasing the sensitivity level, a lower specificity level, i.e., lower specificity, is caused, and thus it remained impossible to have an improvement of the accuracy. Accordingly, as shown in Table 5, the inventors of the present invention handled plural tumor markers having relatively low specificity, and found an effective complex marker. They directed their attention to CEA $\times$ TPA, FT/Fe, and also combination thereof, for example. The table is related to examination in early colon cancer and lung cancer. It is noted by the inventors that, with a single tumor marker like CEA and TPA, it remained impossible to enhance both the sensitivity and specificity, but, by increasing the combination of a complex marker, their level and precision level of the diagnosis accuracy can be enhanced.

[Table 5]

| | Tumor Marker | sensitivity(%) | specificity(%) | accuracy(%) |
|---|---|---|---|---|
| (1) | Mayo Clin.* | | | |
| | CEA ($\geqq$4.4ng/ml) | 17.5(14/80) | 98.5(128/130) | 67.8(142/210) |
| | TPA ($\geqq$125U/L) | 37.5(30/80) | 83.1(108/130) | 65.7(138/210) |
| | FT/Fe ($\geqq$0.4) | 27.5(22/80) | 69.2(90/130) | 53.3(112/210) |
| | TPA$\times$CEA($\geqq$380) | 28.8(23/80) | 99.2(129/130) | 72.4(152/210) |

(continued)

| Tumor Marker | sensitivity(%) | specificity(%) | accuracy(%) |
|---|---|---|---|
| TPA×CEA/(FT/Fe)(≧600) | 31.3(25/80) | 91.5(119/130) | 68.6(144/210) |
| TPA×CEA≧380 and/or TPA×CEA/(FT/Fe)≧600 | 42.5(34/80) | 90.8(118/130) | 72.4(152/210) |

*The number of cases; colon cancer(early stage), 60; lung cancer(early stage), 20; benign colon, 45; benign lung, 15; normals, 70.

As shown in Fig. 17, early cancer and benign tumor (benign disorder) can be discriminated from each other in people having early colon cancer, people having benign colon disorder, and healthy people. That is because, as shown in the graph illustrating the positive percentage of each tumor marker in Fig. 16, the inventors of the present invention found a complex marker for discriminating benign tumor (benign disorder) from healthy state (health people). As such, it becomes possible to achieve the classification by discriminating cancer from healthy state (health people) as a first step and then, as a second step, by using a complex marker (Fe/SA), to discriminate cancer from benign tumor (benign disorder) . It is noted that the discrimination can be achieved by, to avoid the cause complications by an inflammatory disease, measuring both the C reactive protein value (C inflammatory protein value, CRP value) and sialic acid value during the evaluation of $\alpha$1-globulin fraction and subtracting the part contributed by the inflammation.

The inventors of the present invention also noted that, for cancer with cell number of $10^9$ or more, analysis based on blood serum protein fraction is used for screening (identifying and diagnosing) cancer. This diagnosis method of cancer screening using blood serum protein fraction can be carried out at low cost, and it is also convenient and easily employable by a clinician. It is considered that the life of cancer can be classified by using combination of a tumor marker for cancer of "preclinical cancer stage", incorporating blood serum protein fraction analysis for cancer of "clinical cancer stage", and using both of them in combination.

The present invention is devised to solve the problems described above. Namely, an object of the present invention is to provide a method for classifying the life of cancer to contribute to prevention and treatment of cancers by not only finding early cancers in specific organs according to examining the existence of minute cancer in any part of a human body and simultaneously carrying out an ALP isozyme pattern analysis and a tumor marker analysis, or an analysis of blood serum protein fraction followed by overall evaluation, but also identifying a high risk group for minute cancers present at any parts and early cancers of clinical cancer stages and classifying the stages of progressed cancers, and to suggest a scientific way for coping by precisely evaluating the progress of a treatment for a cancer patient.

In other words, it is aimed to provide a data collection method to be used for classifying classifiable cancer life in which, by carrying out a determination to discriminate cancer from health state during the first step and a determination to discriminate cancer from benign tumor during the second step, both the minute cancer at a preclinical cancer stage and also the clinical cancer stage in the life of cancer are detected, and the risk of them are accurately determined.

Solution to Problem

One embodiment of the present invention is a data collection method to be used for classifying cancer life to discover development of minute cancer occurring in a human body and to perform data analysis of risk of tumor and risk of cancer in two divided stages of a preclinical cancer stage and a clinical cancer stage, wherein,
for the data used for analysis of a preclinical cancer stage,
in order to collect data of an occurrence and a ratio of ALP I and activity value of ALP II and ALP III from patterns of the ALP I to the ALP IV as the ALP isozyme, examine the ratio of the numerical data, and perform data analysis for proliferation activity of cancer cells in view of APA calculated from ALP isozyme angle showing sharpness of the ALP II and the ALP III, and
additionally, for avoiding a cause contributed by an inflammatory disease, to perform an analysis while subtracting numerical data of a related part also occurring in the inflammatory disease from each numerical data obtained by measuring both the C reactive protein value (C inflammatory protein value, CRP value) and sialic acid value so as to perform data analysis for the existence and proliferation status of occurring minute cancer, and
for the data used for analysis of a clinical cancer stage,
to collect data from a changed state like a decrease in albumin fraction and an increase in $\alpha$1-globulin fraction, $\alpha$2-globulin fraction, and $\gamma$-globulin fraction that are shown in a protein fraction image and perform an analysis for each data, and
additionally, for avoiding a cause contributed by an inflammatory disease, during the data analysis of protein fraction image, to perform an analysis while subtracting numerical data of a related part also occurring in the inflammatory disease from each numerical data obtained by measuring both C reactive protein value (C inflammatory protein value, CRP value) and sialic acid value so as to perform data analysis for carrying out evaluation of the risk of tumor and the risk of cancer at several steps,
blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, and the data relating to the mobility, chromosome shape, and density of each isozyme are collected by matching a protein fraction image against the ALP isozyme.

Advantageous Effects of Invention

According to the data collection method used for classifying a preclinical cancer stage as one embodiment of the present invention, since there is close relationship between cancer cell proliferation and APA, by analyzing the ALP isozyme pattern using each of those collected data, minute cancer with cell number of $10^4$ to $10^9$ can be detected. Frequent appearance of the ALP I and also an increase in the ALP I in accordance with a progress of cancer can be determined. The ALP II is referred to as systemic ALP, and it is recognized to be involved with the ALP in every organ. It is known that, in a case in which the total activity of the ALP has increased, an increase in the ALP III is shown in osteogenesis, liver cirrhosis, chronic kidney failure or the like. If the ALP is within a normal range, the ALP III increases in case of having new cell generation, for example, proliferation of minute cancer, regeneration of liver, and clinical cancer with slow progress.

Furthermore, according to the data collection method used for classifying a preclinical cancer stage as one embodiment of the present invention, as the progress of cancer accelerates in clinical cancer, the ALP III is changed to the ALP II according to an action of neuraminidase present in blood, thus yielding reduced ALP III. It is highly likely that the ALP III is a modification enzyme which has a dephosphrylating activity related to the proliferation of newly generated cells.

According to the data collection method used for classifying a preclinical cancer stage as one embodiment of the present invention, based on an analysis of reconstituted pattern of each the ALP isozyme, the ALP II (ALP 2), the ALP III (ALP 3), and the ALP IV (ALP 4) can be identified with high precision and high rate. In particular, although the ALP IV is detected at high rate from cancer tissues, appearance frequency of the ALP IV in blood serum of a cancer patient is said to be 1 to 30%, and, due to the low activity, it is simply not observed or mistaken as the ALP III. According to the analysis of a reconstituted pattern of the present invention, it is possible to collect data from which the ALP IV can be detected at high rate.

According to the data collection method used for classifying a preclinical cancer stage as one embodiment of the present invention, when identification of the ALP isozyme pattern is not clear, by classifying the tumor stage based on a tumor marker and introducing a model for stage classification, data allowing accurate evaluation of the cancer development stages including minute cancer to clinical cancer can be collected.

As described above, in each embodiment of the present invention, by using each data collected by the data collection method, it is shown that the ALP isozyme I appears when there is an increase in total activity such as primary liver cancer, liver invasion, stasis liver, or fatty liver, or the like, the ALP II referred to as hepatic ALP is also recognized from pericardial water, an increase in the ALP III is shown in osteogenesis, liver cirrhosis, chronic kidney failure or the like when total ALP activity increases, and there is a close relationship between cancer cell proliferation and the pattern of the ALP isozymes including the ALP I to the ALP IV, and thus, according to analysis of the pattern of those ALP isozymes, minute cancer with cell number of $10^4$ to $10^9$ can be detected and also risk of the minute cancer can be evaluated.

Furthermore, there is an excellent effect that, when identification of the ALP isozyme pattern is not clear, by classifying the tumor stage based on a tumor marker and introducing a model for stage classification, data allowing accurate evaluation of the cancer development stages including minute cancer to clinical cancer can be collected.

Brief Description of Drawings

Fig. 1 is an explanatory drawing illustrating the life of cancer, which is classified into 3 stages including "precancer stage", "preclinical cancer stage", and "clinical cancer stage", and the correlation with a size of cancer cell.
Fig. 2 is a flowchart representing a method for determining "preclinical cancer stage" and "clinical cancer stage" according to the data collection method used for classifying the life of cancer as one embodiment of the present invention.
Fig. 3 is a graph showing the pattern of the ALP I to the ALP IV as an ALP isozyme according to the data collection method used for classifying a preclinical cancer stage of Example 1.
Fig. 4 is a graph and a table showing the pattern of the ALP I to the ALP IV as an ALP isozyme according to the data collection method used for classifying apreclinical cancer stage of Example 1, in which (a) is an ALP isozyme pattern of a 60-year old man, before and after the operation of colorectal cancer, and (b) relates to the ALP isozyme of a patient with esophageal cancer.

Fig. 5 is a graph and a table showing the pattern of the ALP I to the ALP IV as an ALP isozyme according to the data collection method used for classifying apreclinical cancer stage of Example 1, in which (a) shows a change in the marker and isozyme in a 33-year old woman with breast cancer (about 1 g), before and after simple mastectomy, and (b) relates to the ALP isozyme of the same patient, before and after the second operation.

Fig. 6 is a graph showing the pattern of the ALP I to the ALP IV as an ALP isozyme in the heat treatment and reconstitution experiment for each isozyme according to the data collectionmethod used for classifying apreclinical cancer stage of Example 1.

Fig. 7 includes (a) in which a change over time of the tumor marker in blood serum of a patient with gall bladder cancer at stage IV (female, 50-year old) and (b) in which the time course value of Δferritin is classified into three types, i.e., increasing type, constant type, and decreasing type according to the data collection method used for classifying a preclinical cancer stage of Example 1.

Fig. 8 includes the increasing type (a) and decreasing type (b) of Δferritin according to the data collection method used for classifying a preclinical cancer stage of Example 1.

Fig. 9 is an explanatory drawing illustrating the 5-step evaluation method based on cancer growth process and the ALP isozyme.

Fig. 10 is a graph illustrating the classified protein fraction name, result, unit, and reference values according to the data collection method used for classifying the clinical cancer stage of Example 2.

Fig. 11 is a protein fraction analysis drawing in which the classification is made according to the data collection method used for classifying the clinical cancer stage of Example 2.

Fig. 12 is a distribution diagram illustrating the distribution state of a product value of blood serum $\alpha$1-globulin $\times$ $\alpha$2-globulin in early colon cancer when the classification is made according to the data collection method used for classifying the clinical cancer stage of Example 2, in which the upper column represents the colon cancer (40 cases), the middle column represents benign tumor (30 cases), and the bottom column represents a healthy subject (50 cases).

Fig. 13 is a distribution state of a product value of blood serum TPA $\times$ $\alpha$1-globulin in early colon cancer when the classification is made according to the data collection method used for classifying the clinical cancer stage of Example 2, in which the upper column represents the early colon cancer (40 cases), the middle column represents benign tumor (30 cases), and the bottom column represents a healthy subject (50 cases).

Fig. 14 is a distribution diagram examined against a product value of $\alpha$1-globulin $\times$ $\alpha$2-globulin in various cancer cases.

Fig. 15 is a distribution diagram examined against a product value of TPA $\times$ $\alpha$1-globulin in various cancer cases.

Fig. 16 is a graph representing the positive percentage of each tumor marker in people having early colon cancer, people having benign colon disorder, and health people.

Fig. 17 is a graph representing the positive percentage of each tumor marker in people having early colon cancer, people having benign colon disorder, and health people.

Description of Embodiments

The data collection method used for classifying the life of cancer as one embodiment of the present invention is a method for collecting data for classifying the tumor risk and cancer risk into 1 to 4 stages by identifying a change like a decrease in albumin fraction, or an increase in $\alpha$1-globulin fraction and $\alpha$2-globulin fraction, $\gamma$-globulin fraction that are shown on a protein fraction image when classification of cancer at a clinical cancer stage is made.

Example 1

Hereinbelow, the embodiments of the present invention are explained in view of the drawings.

<Method for collecting data used for classifying "minute cancer at a preclinical cancer stage" from life of cancer>

Hereinbelow, a preferred embodiment of the method for collecting data used for classifying the life of cancer of the present invention is explained in view of the drawings.

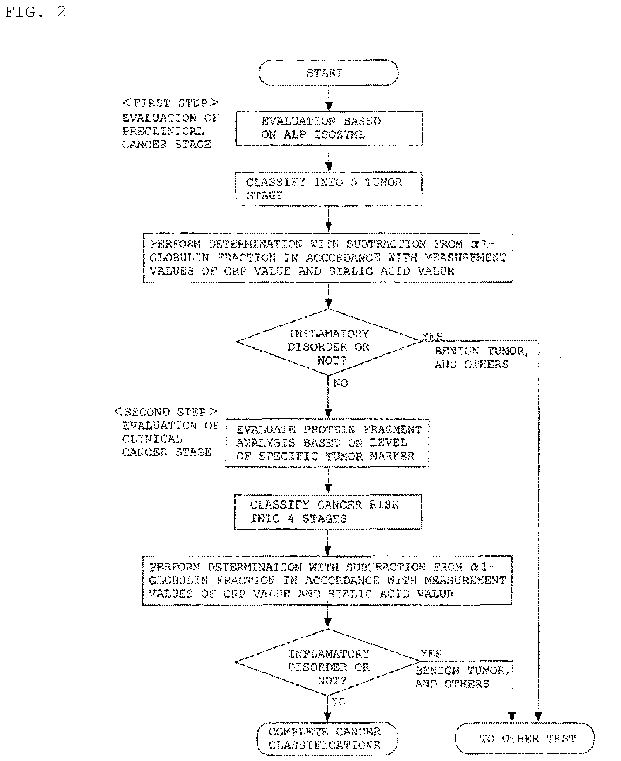

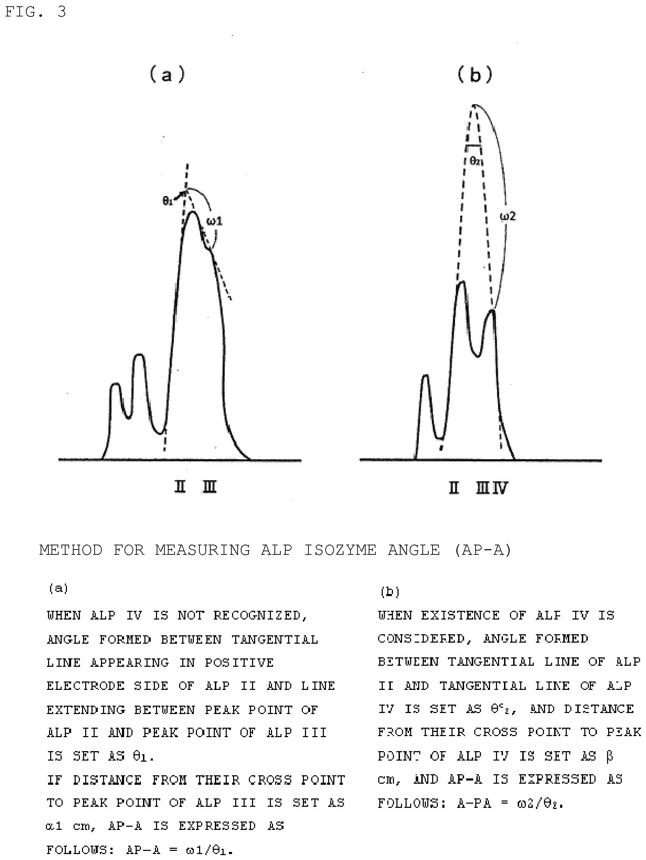

Fig. 1 is an explanatory drawing illustrating the life of cancer, which is classified into 3 stages including "precancer stage", "preclinical cancer stage", and "clinical cancer stage", and the correlation with a size of cancer cell. Fig. 2 is a flowchart representing a determination method using data collected from a "preclinical cancer stage" and a "clinical cancer stage" according to the data collection method used for classifying the life of cancer as one embodiment of the present invention. Figs. 3 to 8 show the pattern of the ALP I to the ALP IV as an ALP isozyme according to the data collection method used for classifying a preclinical cancer stage of Example 1.

The cancer classification method using the ALP isozyme relating to the data collection method used for classifying the preclinical cancer stage of Example 1 of the present invention is a method for biochemical detection of minute cancer (including tumor marker induction) by utilizing, as an index of differentiation caused by the existence of minute cancer, the alkaline phosphatase (ALP) isozymes and $\Delta$CEA or $\Delta$ferritin (change per unit hour), and having the classification of minute cancer. The ALP is widely present in a living human body, and the activity of the ALP increases in any one of the following cases: energy metabolism, differentiation of leucocytes or mammary gland; porosis; and, connective tissues under regeneration. Due to this, it is considered that the ALP plays an important role in canceration, in which the canceration is regarded as a kind of dedifferentiationor abnormal differentiation. In other words, in a condition in which the total activity of the ALP in the blood serum is within a normal range of value, there is an intimate relationship between the proliferation of the cancer cells and the patterns of the isozymes ranging from the ALP I to the ALP IV. For example, it is assumed from a pathological point of view that the cell number of $10^6$ is a biological limit (reversible stage). According to the method for classifying a preclinical cancer stage of Example 1, minute cancer having cell number of from $10^4$ to $10^9$ (i.e., in weight, from 10 $\mu$g to 1 g) is detected through analysis of both a change ($\Delta$) of the tumor marker in a predetermined period of time and the isozymes. The minute cancer is considered to be at a reversible stage, and clinical cancers having a weight of equal to or more than 1 g are considered to be a non-reversible stage.

For the sake of convenience, detectable minute cancers are classified into micro-cancers, which are at the level of micrograms and have cell number of from $10^4$ to $10^6$, and, milli-cancers, which are at the level of milligrams and have cell number of from $10^6$ to $10^9$. The method for evaluating cancer using the ALP isozyme of Example 1 of the present invention is a method in which blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, the mobility, chromosome shape, density, and the like of each isozyme are determined by matching the protein fraction image against the ALP isozyme, development of a minute cancer that is occurring is discovered, and also the risk of the cancer is classified.

According to the method for classifying cancer using the ALP isozyme relating to the data collection method that is used for classifying a preclinical cancer stage of Example 1, proliferation activity of cells of the cancer is analyzed overall by using values of APA, in which the values are calculated with reference to occurrence of the ALP I and a ratio of the occurrence in the ALP isozymes, activity ratio of the ALP II to the ALP III, and, ALP isozyme angle showing sharpness of both the ALP II and the ALP III in patterns of the ALP I to ALP IV isozymes, whereby the existence and proliferation state of the occurring minute cancer are estimated.

<Classification method>

To see whether or not the minute cancer occurs in any part of in a human body, patterns of $\Delta$CEA and $\Delta$ferritin and also the ALP isozymes are analyzed and overall determination thereof is carried out.

(1) For quantification of $\Delta$CEA, an immunoenzyme assay (Abott) allowing quantification of a trace amount was used. Although the CEA remarkably varies among individual subjects, it is well known that, in an individual subject, the CEA varies in a range of equal to or less than 1.0 ng/ml for a long period of time. Herein, a change amount between as a value of the CEA at a predetermined time and $\alpha_2$ as a value of the CEA at a time after the lapse of a certain period of time is defined as follows: $\Delta$CEA = $\alpha_2$ - $\alpha_1$. When the $\Delta$CEA is equal to or more than 0.4 $\pm$ 0.1 ng/ml, it may be considered to a variable width in significant sense.

(2) As for the $\Delta$ferritin, ferritin originating from liver was used in the embodiments of the present invention. For example, a change amount between $\beta_1$ as a value of ferritin at a predetermined time and $\beta_2$ as a value of ferritin at a time after the lapse of a certain period of time is defined as follows: $\Delta$ferritin = $\beta_2$ - $\beta_1$. When the change amount is equal to or more than 4 $\pm$ 1 ng/ml, possibility of the existence of the minute cancer is presumed, provided that: in the case of anyone having substantial liver failure, substantial pancreas failure, or hemochromatosis, a value of the ferritin increases. Meanwhile, since a value of the ferritin decreases in the case of iron-deficiency anemia, additional consideration is required.

Fig. 3 is a graph showing the pattern of the ALP I to the ALP IV as an ALP isozyme according to the data collection method used for classifying a preclinical cancer stage of Example 1.

(3) Method for overall determination of ALP isozyme

The patterns of the ALP isozymes are determined overall based on the following factors:

1) Occurrence of the ALP I and ratio thereof.
2) Activity ratio of the ALP II to the ALP III is determined.
3) ALP isozyme angle (AP-A), which represents a sharpness of the ALP II and the ALP III.

The AP-A is measured as follows (see, Fig. 3). An angle formed between a tangential line appearing in the positive electrode side of the ALP II and a line extending between a peak point of the ALP II and a peak point of the ALP III is set as $\theta°_1$. If the distance from the cross point of those two tangential lines to a peak point of the ALP III is set as $\omega 1$ cm, AP-A is expressed as follows: $\omega 1/\theta_1$. When it is believed there is an occurrence of the ALP IV, if the distance from the cross point with the tangential line of the ALP III to a peak point of the ALP III to the ALP IV is set as $\omega 2$ cm, AP-A is calculated as follows: $\omega 2/\theta_2$.

Furthermore, according to the method for classifying cancer using the ALP isozyme relating to the data collection method that is used for classifying a preclinical cancer stage of Example 1, there is a phenomenon also occurring in an inflammatory disease, and thus the determination needs to be made with exclusion of a part contributed by an inflammatory disease. As such, by also measuring a sialic acid value with C reactive protein value (C inflammatory protein value, CRP value), more accurate cancer classification can be achieved.

For example, depending on the height of the measured C reactive protein value (C inflammatory protein value, CRP value), the determination is made after subtracting the part contributed by the C reactive protein value from $\alpha 1$-globulin fraction. The determination is made by subtracting the level contributed by sialic acid measurement value (contribution level).

Next, examples of the data collection method that is used for classifying the life of cancer of the present invention are explained.

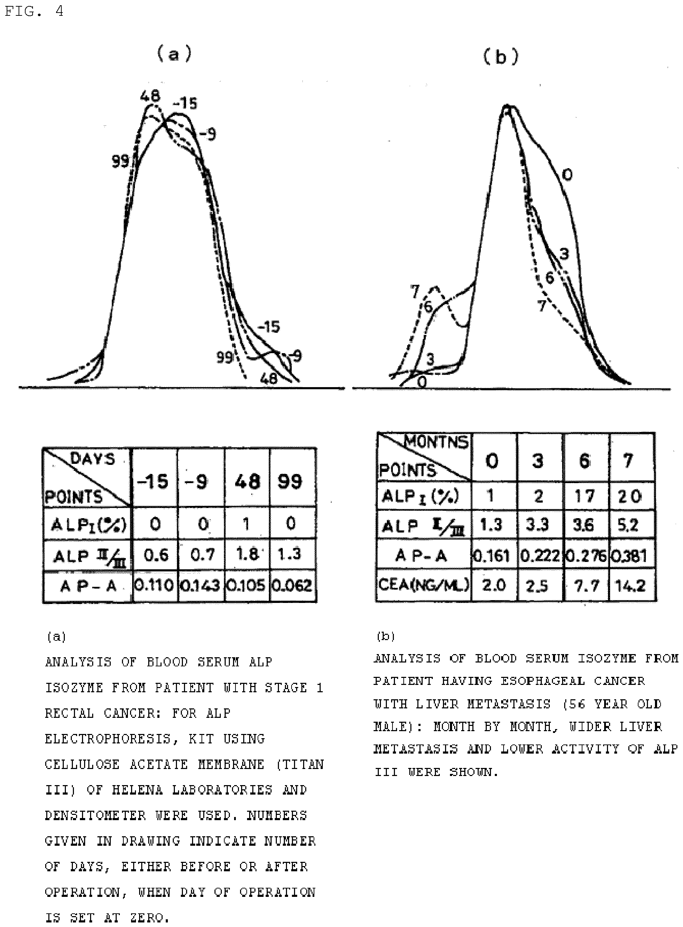

Fig. 4 is a graph and a table showing the pattern of the ALP I to the ALP IV as an ALP isozyme according to the data collection method used for classifying apreclinical cancer stage of Example 1, in which (a) shows an ALP isozyme pattern of a 60-year old man, before and after the operation of colorectal cancer, and (b) shows a Zymogram of the ALP isozyme of a patient with esophageal cancer.

Case (1)

A 60-year old man had his colorectal cancer (stage 1 "pmN$_0$P$_0$H$_0$") treated through a Miles' operation. The date of such operation was set at "zero" and the patterns of the ALP isozymes before and after the operation were analyzed according to the method of the overall determination (Fig. 4-(a)). 1% of the ALP I still remained on Day 48 from the operation, but it disappeared on Day 99 from the operation. A ratio of the ALP II to the ALP III before the operation shows reversed patterns of the ALP II and the ALP III, yielding a value of equal to or less than 1.0. On the other hand, after the operation, the ALP III started to decrease, and on Day 48 and on Day 99, it shows a value of to 1.8 and 1.3, respectively, within a normal range of values (1.6 $\pm$ 0.4). Further, as to the AP-A, although it gradually became worse before operation, it was recognized that the AP-A falls within a normal range of values (equal to or less than 0.1 $\pm$ 0.01) on Day 48 after the operation and also thereafter. Although several days were required before the AP-A is recovered to be within a normal range after the operation, it is considered that the minute cancers, which remained in a peripheral area of the removed colorectal cancer, required such a period of time before showing gradual shrinkage.

Case (2)

In Fig. 4-(b), the ALP zymogram of a patient having esophageal cancer (which has metastasized to the liver) is shown. The total activity of the ALP was within a normal range during the examination period. The cancer has gradually developed month by month, and thus an increase in the value of the CEA was shown (Fig. 4-(b)). A gradual increase in the ALP I, the ALP II/III, and the AP-A was also recognized.

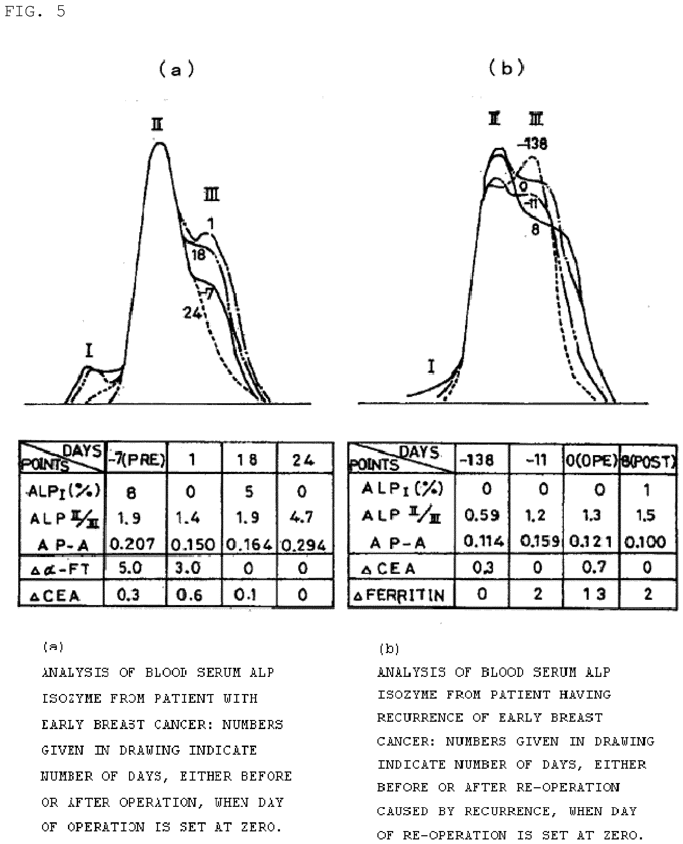

Fig. 5 is a graph and a table showing the pattern of the ALP I to the ALP IV as an ALP isozyme according to the data collection method used for classifying apreclinical cancer stage of Example 1, in which (a) shows a change in the marker and isozyme in a 33-year old woman with breast cancer (about 1 g), before and after simple mastectomy, and (b) relates to the ALP isozyme of the same patient, before and after the second operation.

Case (3)

Variations in each of the markers and the ALP isozymes found in a 33-year old female having breast cancer (approximately 1 g) before and after a simple mastectomy were examined (Fig. 5- (a)). The $\Delta$ values in the table correspond to the $\alpha$-FP after subtracting 2 ng/ml therefrom, and the CEA after subtracting 1.1 ng/ml therefrom (2 ng/ml and 1 ng/ml are the minimum value in the period of time). After the above surgical operation, an ITC therapy was conducted two times only. It is considered that Fig. 4- (a) illustrates a process in which one minute cancer dispersedly present in a peripheral area of the surgery site gradually grows . The ALP isozymes have a speedy reaction, while it is considered that each of the $\alpha$-FP and the CEA seems to react with a slight time lag. Particularly, the characteristic pattern showing a decrease in the ALP III can be well grasped numerically by using the AP-A. Although the AP-A decreased slightly due to the operation, it was observed that the AP-A rapidly increased again with some time lag, and on Day 24 after the operation, it reached a value of 0.294, which indicates a possibility of recurrence.

Next, nine months after the operation, the cancer of about 0.5 g has occurred again in the vicinities of the operation site of the same patient, and it was removed accordingly. An oriental-medicine therapy and basic therapy were initiated one week before the operation. In Fig. 4-(b), the analysis of the ALP isozymes before and after the second operation was shown. Numbers in the drawing indicate the number of days having counted from the date of the operation, in which the date of the operation was set at "zero". ΔCEA represents a value resulting from subtracting 1.7 ng/ml and the Δferritin represents a value resulting from subtracting 12 ng/ml. It is evident that both the ferritin and the CEA increase up to a date immediately before the operation.

It is suggested that, through analysis of the ALP isozymes in the blood serum found in the patient suffering from the cancers before and after the operation, there is an intimate relationship between the proliferation activity of the cancer cells and an occurrence of the ALP I, in particular, and a change in pattern of each of the ALP II and the ALP III. By checking a change of the tumor markers over the time, and, further, by combining such check with the analysis of the ALP isozymes, it is considered that both the existence and the proliferation status of the minute cancers can be estimated.

Next, the analysis employed for the method that is used for classifying a preclinical cancer stage of Example 1 will be described. Under current circumstances that each of the isozymes ranging from the ALP II to the ALP IV cannot be precisely identified, in order to conduct more accurately the analysis of their patterns, a heat treatment and reconstitution experiments of each of the isozymes were conducted.

<Materials and Methods for Experiment>

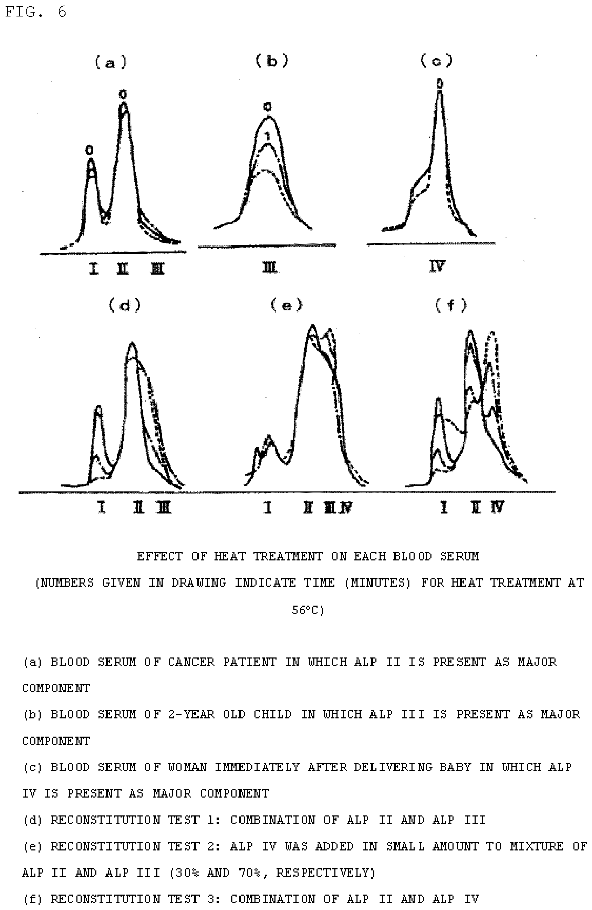

Fig. 6 is a graph showing the pattern of the ALP I to the ALP IV in the heat treatment and reconstitution experiment for each isozyme according to the data collection method used for classifying a preclinical cancer stage of Example 1.

Used in the experiments were a blood serum found in a cancer patient, in which the ALP II is present as a major component due to a remarkable reduction of the ALP III (hereinbelow, abbreviated as the "blood serum 2") ((Fig. 6(a)-0)); a blood serum found in a two-year old child, in which the ALP III is present as a main component ("blood serum 3") ((Fig. 6(b)-0)) ; and, a blood serum (found in a pregnant woman immediately after a delivery of her baby, in which the ALP IV is present as a main component ("blood serum 4") ((Fig. 6(c)-0)).

<Results of Experiment>

Effects of the heat treatment on each of the blood serum. Figs. 6(a), 6(b), and 6(c) relate to the isozyme in which each blood serum is subjected to a heat treatment for 10 minutes at 56°C (in the drawings, the numbers indicate a period of time during which the heat treatment was performed) . The ALP II is considerably stable against the heat at 56°C for 5 minutes, and shows a sharp peak (Fig. 6-(a)).

The ALP III was significantly inhibited by a heat treatment for 10 minutes at 56°C, showing a heat labile property (Fig. 6-(b)). Furthermore, the smooth curve of the ALP III suggests the existence of a wider variation in molecular type of the ALP III. As for the ALP IV, it is shown to be a heat tolerant type, and, its sharp peak clearly indicates the characteristic derived from late-stage placenta (Fig. 6-(c)).

By focusing on the reconstitution experiment 1 (Fig. 6(d)), blood serums in which blood serum 2 and blood serum 3 are combined at various ratios were electrophoresed and analyzed. Through calculation of the activity of each of the ALP II and the ALP III on the basis of the total activity, a ratio of each of the activity was determined. When the ALP III was gradually increased to 1, 25, 75 and 92%, the ALP II is gradually reduced to 99, 75, 25 and 8% in accordance with the increase. In conjunction with the increase in the ALP III fraction, peaks of the ALP II, III showed a round curve, thus forming an obtuse angle.

In a reconstitution experiments 2 (Fig. 6(e)), the ALP I was ignored in the experiment. The blood serum 2 and the blood serum 3 were mixed with each other, and patterns of the isozymes containing a 30% of the ALP II and a 70% of the ALP III were obtained (indicated by "0" in the drawings). These patterns were the same patterns as those obtained from a healthy adult. In a condition in which a ratio of the blood serum 2 to the blood serum 3 was kept constant, the blood serum 4 was gradually added to the mixture followed by analysis. As the ratio (%) of the ALP IV increases, the graph became to have a shoulder part which was then transformed into a pattern having two peaks. When compared to the reconstitution experiment 1 (Fig. 6(a)), it was recognized to have an isozyme pattern having increased ALP IV in a condition in which a sharp shoulder part was formed in the peak of the ALP III, or, the two peaks appeared in the graph.

The reconstitution experiment 3 (Fig. 6(f)): In this experiment, determination was made to see a change of the patterns of the isozymes in accordance with a change in the ratio of the ALP II to the ALP IV. The patterns of the ALP II and the ALP IV were transformed into pattern with a sharp and deep V-shaped two-peaks when the ALP IV is 25 to 75%.

From the analysis of the reconstituted pattern of each of the ALP isozymes, it is possible to identify each of the ALP II, the ALP III and the ALP IV in a simple manner. Since the cancers have a wide variation in molecular types, a detection rate of the ALP of a carcinoembryonic type is poor when a checking process of such ALP relies on a heat treatment only. Heretofore, in spite of a high-detection rate of the ALP IV in the cancer tissues, it is said that an occurrence rate of the ALP IV in the blood of a patient suffering from the cancers is within a range of from 1 to 30%. As for the reason why a detection-rate of the ALP IV in the blood is low, it is considered that, there is a high possibility of some connection with a leakage of the ALP IV into the blood, and, the existence of the ALP IV is either overlooked or mistaken as the ALP III due to the poor activity of the ALP IV. Thus, the ALP IV can be detected at higher rate if the search is made with reference to the patterns of the isozymes that are obtained through the above reconstitution experiments . The ALP I occurs when the total activity found in the cancer cells of primary liver cancer, liver invasion, stasis liver, or fatty liver, or the like increases. However, the inventors of the present invention have found out that, even when the ALP is within a normal range of values, the ALP I of the carcinoembryonic type is often shown, and, the ALP I increases in accordance with a progress of the cancer (Fig. 6(b)).

Meanwhile, the ALP II is called systemic ALP, and thus considered to be a basic ALP. It is known that, in the case in which the total activity of the ALP has increased, an increase in the ALP III is shown in osteogenesis, liver cirrhosis, chronic kidney failure or the like. If the ALP is within a normal range, the ALP III increases in case of having new cell generation, for example, proliferation of minute cancer, regeneration of liver, and clinical cancer with slow progress. When the progress of cancer is accelerated in clinical cancer, the ALP III decreases, probably, due to the exhibition of neuramidinase activity in the blood or, transformation of the ALP III into the ALP II. As for the ALP III, there is a high possibility that the ALP III is a modification enzyme having a dephosphorylation activity that is associated with the proliferation of new-generated cells. Neuraminidase is an enzyme for releasing sialic acid. It is also referred to as a sialidase, and is an asialoprotein for dissociating sialic acid (neuraminic acid) from an oligosaccharide.

It becomes possible to estimate the existence of the minute cancers by diagnosis using the tumor markers such as alkaline phosphatase (ALP) isozymes found in the blood serum, $\Delta$CEA, and $\Delta$ferritin (i.e., difference per predetermined period of time) . However, although it is said that an occurrence rate of the ALP IV in the blood serum is within a range of from 1 to 30%, the detection rate of the ALP IV in the cancerous tissues is considerably high. It is expected in this regard that, even after being produced in cancer cells, for the tumor markers to enter the blood stream, a certain mechanism needs to be involved therewith. Consequently, in order to confirm the existence of a minute cancer, the inventors of the present invention carried out, on the basis of the fact that vitamin A and heat are known to be effective for increasing the tumor markers of cultivated cells in vitro, an induction process of tumor markers originated from possible cancerous tissues for increasing the leakage of such markers into blood, in which the induction process was conducted by using vitamin A and also by hyperthermia having an effect of heating a deep area of a human body with a use of far infrared radiation.

<Methods>

As a subject, fifteen volunteers (4 males and 11 females) were employed. All the volunteers were considered to be sound in health and free from any cancer. Their ages were within a range of from 28 to 38. As an example of the clinical cancers, a patient having gall bladder cancer at stage IV (45-year-old, female) was selected. As an induction process, vitamin A (retinol palmitate) was administrated at a dose of 50000 I.U. through intramuscular injection. After one hour, the testee was treated by hyperthermia at a temperature of 56°C for a period of 20 minutes with the use of far infrared ray.

<Results>

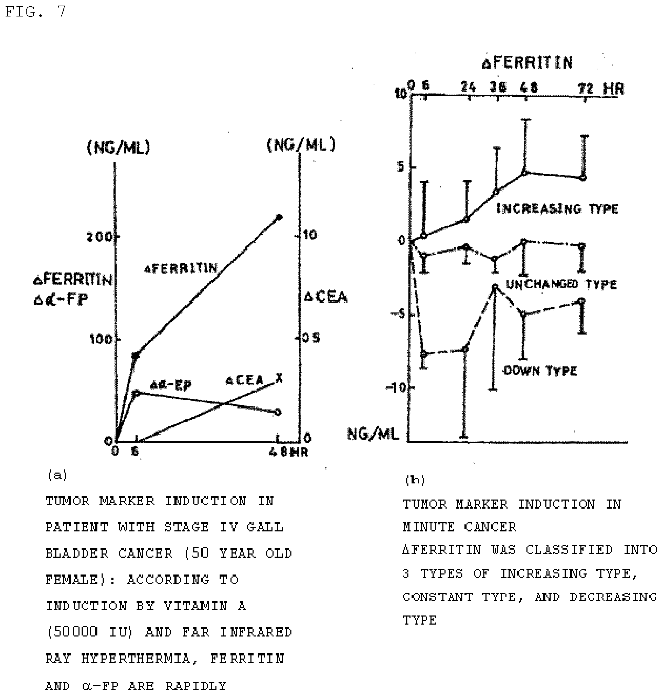

Fig. 7 includes (a) in which a change over time of the tumor marker in blood serum of a patient with gall bladder cancer at stage IV (female, 50 year old) and (b) in which the time course value of $\Delta$ferritin is classified into three types, i.e., increasing type, constant type, and decreasing type according to the data collection method used for classifying a preclinical cancer stage of Example 1.

The patient suffering from gall bladder cancer was subjected to the induction process using the tumor markers, and then a change of the tumor marker found in the blood serum over time was examined (Fig. 7-(a)). It was recognized that there is a rapid leakage of ferritin or $\alpha$-FP into blood after a lapse of 6 hours. It was recognized that the ferritin varied with an amount of 213 ng/ml and the $\alpha$-FP varied with an amount of 50 ng/ml.

After 48 hours, the CEA increased only by an amount of 0.3 ng/ml. Then, the induction process was applied to each of the 15 volunteers. At a time point of Hour 6, Hour 24, Hour 36, and Hour 48, and both before the process, the blood was collected, and a difference in blood serum ferritin value between each time point and before the process was set as $\Delta$ferritin, and with the elapsed time, it was categorized into 3 types, i.e., increasingtype, constant type, and decreasing type (Fig. 7-(b)).

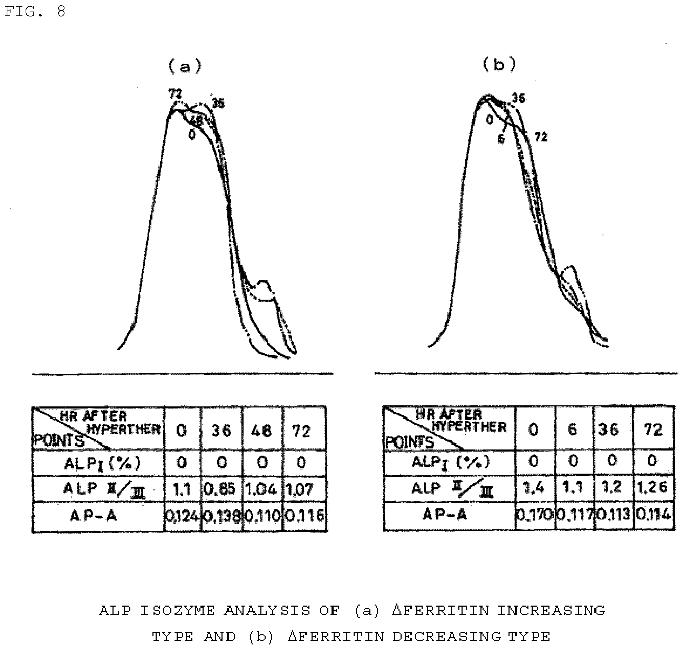

Fig. 8 represents the increasing type (a) and decreasing type (b) of $\Delta$ferritin according to the data collection method used for classifying a preclinical cancer stage of Example 1.

In the case of the constant type (n = 4), variation width was equal to or less than 4 ng. In the case of the increasing type (n = 6), it was observed that: the Δferritin began to increase after a lapse of 24 hours; and, then reached a peak after a lapse of 48 hours, in most cases. The variation width was within a range of from 7 ng to 12 ng. In the case of the decreasing type (n = 3), after a lapse of 6 hours from the induction process, it was recognized that the ferritin rapidly decreased (7.7 ng/ml ± 0.9 ng). At this time, the variation width was within a range of from 7 ng to 13 ng. After that, the variation width was recognized to gradually return to its initial value. Here, analysis of patterns of the ALP isozymes was conducted by using the method of the present invention. In the case of the constant type of the Δferritin, also the activity ratio of the ALP II and the ALP III (ALP II/III) was fully within a normal range, that is, within a range of from 1.6 ± 0.4. Further, the AP-A was also equal to or less than 0.1, and found to be quite normal in value. In this constant type, it is believed that the cancer is before the minute cancer, and the number of its cells was probably equal to or less than $10^4$, which made it impossible for the cancer to be detected, and thus the patient was judged substantially normal. In Fig. 8(a), the analysis of the ALP isozymes having been classified into the increasing type of the Δferritin was shown. Numbers shown in the drawing indicate elapsed time after the induction treatment.

As illustrated in the analysis shown in the drawings, the ALP II/III ratio after a lapse of 36 hours reached an abnormally low value (equal to or less than 1.0), and it was gradually recovered thereafter. Based on those abnormal values of the ALP II/III and AP-A caused by the induction method, the Δferritin increasing type suggests the existence of minute cancer. Finally, with regard to the decreasing type, analysis of the ALP isozyme is shown in Fig. 8 (b). In the case of the decreasing type, the ALP II/III ratio showed an abnormally low value after a lapse of 6 hours, and it was gradually recovered thereafter. The AP-A shows normal values after a lapse of 6 hours or more. On the basis of the variation in accordance with time-based correlation with Δferritin, it is considered that the decreasing type of the Δferritin probably indicates the existence of the minute cancer associated with some inflammations.

As described above, even when the variation is minor, if the ΔCEA varies in a range of a value equal to or more than a value of 0.4 ± 0.1 ng/ml and the Δferritin varies in a range of a value equal to or more than a range of from 4 ng/ml to 7 ng/m as a result of carrying out the induction process, it is necessary to recommend a search for the minute cancers. After that, the analysis of the ALP isozymes is performed to confirm the existence of the minute cancers. Particularly, when existence of a fraction of heat tolerant ALP isozymes still remaining active after the heat treatment for 10 minutes at a temperature of 56°C is checked in combination with a so-called "ultra-micro measurement" with respect to the activity of the ALP (heat treatment conducted at a temperature of 65°C for 10 minutes) by using a fluorescence spectrophotometer and the activity measurement of heat tolerant ALP isozymes, the existence of the minute cancer can be more clearly identified.

As for the clinical cancers, the ΔCEA has a variation width that is equal to or more than 1.0 ± 0.1 ng/ml and the Δferritin has a variation width that is equal to or more than 20 ng/ml. Although only the increasing type is shown in the induction pattern of the clinical cancers in this example, the decreasing type was also very often observed. Each of the increasing type and the decreasing type among the volunteers are considered to be a miniature type of the induction patterns of the clinical cancers. Further examination will be required in future to determine whether a difference in the existence form between the decreasing type and the increasing type is resulted from only the presence or absence of the inflammation or from a difference in existence condition of the cancers. Naturally, in the inflammation of any one of parenchymatous organs in which value of the ferritin increases and the hemochromatosis, examination must be carefully conducted. At this time, analysis of the patterns of LDH isozymes may also provide important information. Further, since a value of the ferritin decreases when iron level is low in blood serum, it is necessary at first to bring the blood serum iron level back to a normal value and then the induction process is conducted. Because the ferritin value tends to be low in case of a cancer in digestive system, a caution is required. Further, starvation often causes a rapid and temporary increase in a value of the ferritin. In the case of a simple inflammation such as a hepatitis and the like, only a value of the ferritin rapidly decreases, and an abnormality in ΔCEA or the ALP isozymes is not recognized.

An increase in Δferritin caused by vitamin A and far infrared hyperthermia was classified into three types, i.e., the increasing type, the decreasing type, and the constant type. The constant type is considered to be a healthy person having not even a minute cancer. When Δferritin is within a range of from 4 ng/ml to 20 ng/ml and the ΔCEA is within a range of from 0.4 ng/ml to 1.0 ng/ml, it was considered that the existence of minute cancer is assumed. Furthermore, in the analysis of the ALP isozymes (within a range in which the ALP activity falls in a normal value range), it is possible to detect more clearly the existence of the minute cancer by using: the ratio of the ALP I, the ALP II/III ratio, and a change in the AP-A.

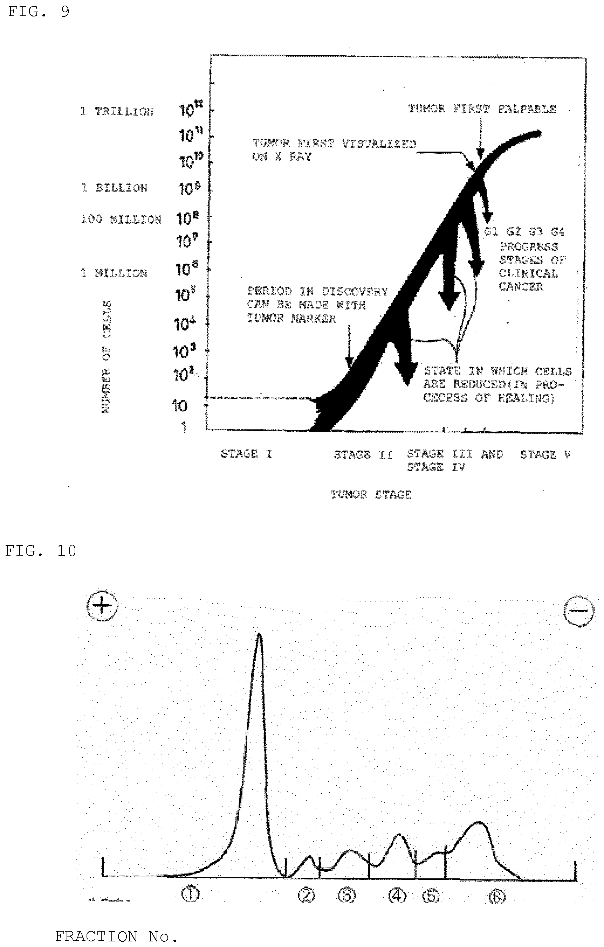

Fig. 9 is an explanatory drawing illustrating the 5-step evaluation method based on cancer growth process and the ALP isozyme.

The growth level of the tumors determined by screening of the tumor markers is classified into several stages in accordance with the degree of growth of the tumors to find out cancers occurred in a normal person in appearance, and also the risk of the cancer is estimated. For example, the above classification of tumor growth level includes stages of two or more levels, in which a state quite free from any minute cancer was defined as a stage I, a precancer state is classified into a stage II and a stage III, a preclinical cancer state is defined as a stage IV, and, a state in which a cancer having a weight of 1 g or more is believed to be present is defined as a stage V.

Example 2

<Method for classifying "cancer at a clinical cancer stage" in cancer life>

Example 2 relates to a method for classifying "cancer at a clinical cancer stage" according to the data collection method that is used for classifying the life of cancer. The data collection method that is used for classifying the life of cancer in Example 2 is a method for screening cancer based on multivariate analysis of a blood serum protein fraction. The screening by the method of Example 2 is a method for leading to suitable therapy based on early discovery of cancer, and it is carried out if there is a method for definite diagnosis using close examination after brief screening. An object of this example is not to perform final diagnosis regarding the presence or absence of a disease or a disorder, but a method to be carried out as a pre-step of a close examination for performing definite diagnosis. Furthermore, also for the method classifying "cancer at a clinical cancer stage" of Example 2, protein fractions are explained by using symbols such as $\alpha$, $\beta$, $\gamma$, or the like. However, they may be used with a meaning that is different from the symbols of $\alpha$, $\beta$, $\gamma$ or the like that are used for the method for classifying "cancer at a preclinical cancer stage" of Example 1.

The method of Example 2 is a determination method at the "clinical cancer stage" shown in the flowchart of Fig. 2. Cancer screening only based on protein fraction using the method of Example 2 is a method in which multivariate analysis is carried out on the basis of the combination of mutual inhibition among albumin [%], $\alpha$1-globulin fraction [%], $\alpha$2-globulin fraction [%] , and $\gamma$-globulin fraction [%] and the risk of tumor and risk of cancer are classified and evaluated.

According to the data collection method used for classification in Example 2, blood serum is applied on a support that has been impregnated in a buffer solution, the support is fractionated by electrophoresis at a predetermined liquid temperature to isolate proteins in the blood serum, the ALP isozyme is detected by color-developing with an ALP isozyme staining solution, the mobility, chromosome shape, density, and the like of each isozyme are determined by matching the protein fraction image against the ALP isozyme, development of a minute cancer that is occurring is discovered, and also the risk of tumor and risk of cancer are evaluated.

According to the data collection method used for classification in Example 2, by identifying a change like a decrease in albumin fraction and an increase in $\alpha$1-globulin fraction and $\alpha$2-globulin fraction, and $\gamma$-globulin fraction that are shown in a protein fraction image, the risk of tumor and the risk of cancer are classified into several stages.

<Explanation of albumin fraction>

According to the data collection method used for classification of a clinical cancer in Example 2, albumin and $\alpha$1-globulin fraction (most of immunosuppressive substances) are used. In particular, $\alpha$1-globulin fraction functions as a favorable parameter. Furthermore, as the change in protein fraction image is a phenomenon which also occurs in an inflammatory disease, it is necessary to avoid a case in which the change is caused by the inflammatory disease. As such, by measuring both the C reactive protein value (C inflammatory protein value, CRP value) and sialic acid value, more accurate cancer classification can be achieved.

For example, in case of liver cirrhosis and chronic hepatitis, there is a tendency that albumin is decreased and $\gamma$-globulin is increased. Albumin is produced in a liver, used for regulation of blood osmotic pressure, and it is a material used for transporting hormones and toxic materials. Albumin has a property that it is present in a decreased amount in case of having any of most common diseases.

<Explanation of globulin fraction>

(1) $\alpha$1-Globulin fraction

An increase in the $\alpha$1-globulin fraction is shown in case of having inflammation, cancer, and acute state of stress and rheumatoid disorder. Included in this fraction are antitrypsin, $\alpha$1 soluble glycoprotein, prothrombin, chymotrypsin, elastase, and protease. The latter 2 enzymes are generated from polymorphonucleus cells which can destroy pulmonary tissues unless inhibited by granular white blood cells, and thus the major role of $\alpha$1 antitrypsin appears to be the protection of pulmonary tissues. AFP is also included in the fraction.

(2) $\alpha$2-Globulin fraction

As for the $\alpha$2-globulin fraction, $\alpha$2-HS globulin fraction, ceruloplasmin, erythropoetin, choline esterase, contact globulin, and $\alpha$2-microglobulin are included in the fraction. Haptoglobin binds to hemoglobin, and according to transport by blood circulation, a non-specific response to stress is increased. Chronic protein deficiency causes an increase in $\alpha$2-microglobulin. Ceruloplasmin is produced in the liver in which iron is added to an oxidative enzyme and iron transport to transferrin is promoted.

$\alpha$2-HS glycoprotein is considered to be a non-specific opsonin.

(3) $\beta$-Globulin fraction

As for the $\beta$-globulin fraction, transferrin, $\beta$ lipoprotein, complements, and hemopexin are included in the fraction. Transferrin is produced in the liver, and it is involved with an organ for transporting iron. In addition, 60% of the fraction are engaged in this mechanism. Lipids play a role of integrating a cellular membrane to a precursor cell, and among steroids and bile acid juice, the largest fraction is a $\beta$ lipoprotein.

Hemopexin is utilized as a hemoglobin molecule for producing heme with iron as a nucleus. Complements indicate a complex system of blood serum proteins working against inflammation.

(4) $\gamma$-Globulin fraction

As for the $\gamma$-globulin fraction, IgA, IgG, and IgM are included in the fraction. In the IgA fraction, anti-toxin, antimicrobial agglutinin, cold agglutinin, and isoagglutin are included. They are related to salivary excretion.

IgG includes an antibody of bacteria, virus, or toxin. This fraction is increased in case of acute and chronic diseases . An increased IgM is shown in many diseases.

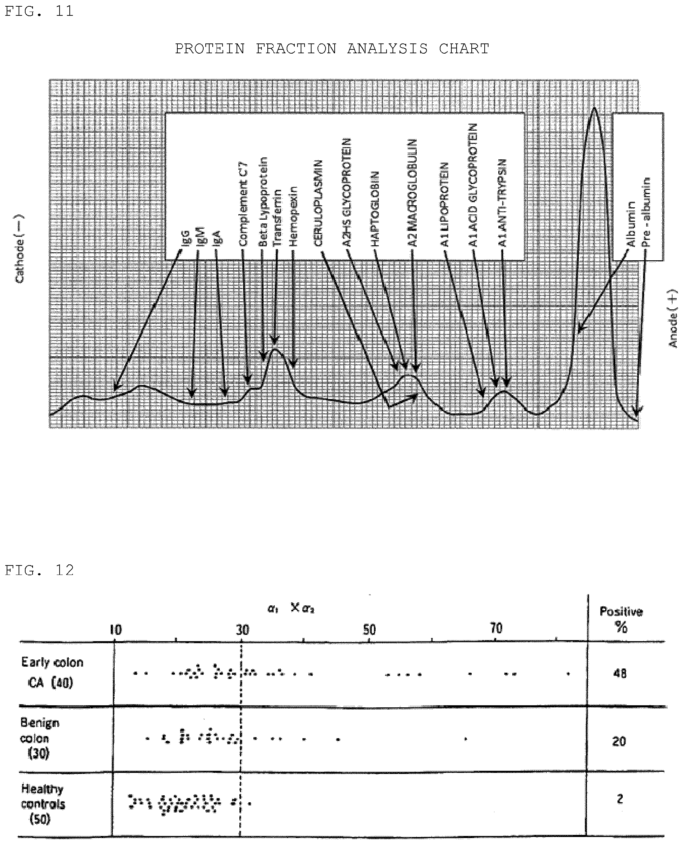

Fig. 10 is a graph illustrating each area ratio by describing the protein fraction name, result, unit, and reference values and adding a check to the change point of each protein fraction. Fig. 11 is a drawing of analyzing each protein fraction.

As for the protein fraction, result, unit, and reference values, as it is illustrated in the drawing and described in Table 1, fraction No. 1 is an albumin fraction (Alb) in which the protein fraction result is 58.7, and the reference value is 55.8 to 66.1.

Fraction No. 2 is an $\alpha$1-globulin fraction ($\alpha$1-G) in which the protein fraction result is 3.1, and the reference value is 2.9 to 4.9.

Fraction No. 3 is an $\alpha$2-globulin fraction ($\alpha$2-G) in which the protein fraction result is 8.7, and the reference value is 7.1 to 11.8.

Fraction No. 4 is a $\beta$1-globulin fraction ($\beta$1-G) in which the protein fraction result is 6.1, and the reference value is 7.7 to 7.2.

Fraction No. 5 is a $\beta$2-globulin fraction ($\beta$2-G) in which the protein fraction result is 3.7, and the reference value is 3.2 to 6.5.

Fraction No. 6 is a $\gamma$-globulin fraction ($\gamma$-G) , in which the protein fraction result is 19.7, and the reference value is 11.1 to 18.8.

[Table 1]

| Test item | | Protein fraction | | | | |
|---|---|---|---|---|---|---|
| Fraction No. | Fraction name | Result | Unit | Reference Value | | |
| ① | Alb | 58.7 | % | 88.8 | ~ | 66.1 |
| ② | $\alpha$1-G | 3.1 | % | 2. 9 | ~ | 4.9 |
| ③ | $\alpha$2-G | 8.7 | % | 7. 1 | ~ | 11.8 |
| ④ | $\beta$1-G | 8.1 | % | 4. 7 | ~ | 7.2 |
| ⑤ | $\beta$2-G | 3.7 | % | 3.2 | ~ | 6.5 |
| ⑥ | $\gamma$-G | 19.7 ↑ | % | 11.1 | ~ | 18.8 |
| | A/G | 1.4 | | 1.3 | ~ | 1.9 |

<Method for classifying into 4 stages>

Based on the results of above Table 1, Fig. 11, and Fig. 12, according to the data collection method used for classifying a clinical cancer stage in Example 2, cancer of 1 gram or more at a clinical cancer stage in the cancer life is classified into 4 stages. For example, the risk of cancer at a clinical cancer stage has four stages as follows:

first stage in which albumin fraction is 65% or more, $\alpha$1-globulin fraction is less than 2.5%, and $\gamma$-globulin fraction is less than 16%,

second stage in which albumin fraction is 60% or more but less than 65%, $\alpha$1-globulin fraction is 2.5% or more but less than 3.0%, and $\gamma$-globulin fraction is 16% or more but less than 20%,

third stage in which albumin fraction is 55% or more but less than 60%, $\alpha$1-globulin fraction is 3.0% or more but less than 4.0%, and $\gamma$-globulin fraction is 20% or more but less than 23%, and

fourth stage in which albumin fraction is less than 55%, $\alpha$1-globulin fraction is 4.0% or more, and $\gamma$-globulin fraction is 23% or more.

Furthermore, those numbers are only exemplifications, and it is not limited to those numbers.

As described above, according to the data collection method used for classifying a clinical cancer stage in Example 2, by adding a biochemical sample of a protein fraction, cancer stage after cancer becomes a clinical cancer, i.e., cancer of 1 gram or more, can be accurately classified. When the cancer stage classification is made according to the classification method of this example, cancer prevention, recurrence prevention, therapeutic effectiveness of an anti-cancer agent, and cancer progress can be numerically analyzed with quite high accuracy. If TMCA test is carried out before and after cancer operation to see whether or not a health food is effective for an individual, an individual has a proper diet, or proper cancer therapy is carried out, it is possible to identify early stage cancer or progressed cancer without having an open surgery. Of course, if TMCA test is carried out after having an operation, it is also possible to clearly determine the success or failure of the operation.

TMCA (tumor marker combination assay) is a method for overall determination of toxicity in a living body (internal environment). This method allows overall risk classification based on determination of toxins (i.e., tumor markers) that are generated from cancer, and utilization of the risk as a health state classification. Originally, this assay has been noted as an only test method for allowing predictive diagnosis of cancer . TMCA method is a method for classifying the cancer risk into 5 stages, i.e., from "clinical cancer" to "ideal health state", only by a simple oriental medicinal test with collection of blood just in an amount of 20 cc.

The health state classification of an internal environment of a living body allows pre-symptomatic-stage-determination of a health state of each individual. It is a very effective method to determine objectively whether or not the life style of each individual including exercise, health food or the like heads toward a healthier direction or a non-healthy direction.

The data collection method used for classifying a clinical cancer stage in Example 2 corresponds to a health barometer, and it allows sure prevention of many misdiagnoses like determination made based on insufficient image diagnosis only.

Furthermore, as the change in protein fraction image is a phenomenon which also occurs in an inflammatory disease, it is necessary to avoid a related part that is caused by the inflammatory disease. As such, by measuring both the C reactive protein value (C inflammatory protein value, CRP value) and sialic acid value, more accurate cancer classification can be achieved.

If the determination is made after subtracting the related part of the C reactive protein value from $\alpha$1-globulin fraction depending on the height of the measured C reactive protein value (C inflammatory protein value, CRP value), and also the determination is made after subtracting the related part of the sialic acid value from $\alpha$1-globulin fraction depending on the height of the measured sialic acid value, it is possible to classify cancer risk while excluding the part contributed by inflammation during evaluation.

The data collection method used for classifying a clinical cancer stage of the present invention can be carried out by general cancer classification. Although the standards may be slightly different in sarcoma or the like, it is also possible to apply the method for classifying a clinical cancer stage of the present invention thereto.

<Test>

Nest, explanations are given for a test example which is based on the data collection method used for classifying a clinical cancer stage of the present invention.

For the data collection method used for classifying the life of cancer of the present invention, several trials were made to enhance the property of discriminating early cancer from benign case using tumor markers. In particular, blood serum protein fractions (immuno-regulatory $\alpha$-globulin and tissue polypeptide antigen (TPA)) are combined, and evaluation was made for the clinical usefulness of a product of $\alpha$1-globulin $\times$ $\alpha$2-globulin and a product of TPA $\times$ $\alpha$1-globulin as a tumor marker. $\alpha$1-Globulin and $\alpha$2-globulin fractions can reflect the proliferation of cancer. Introduction of a multivariate analysis is useful for cancer screening. Furthermore, TPA is known as a tumor-specific and growth-related tumor marker (which reflects cell proliferation ability), which is contained in various cancer tissues and released into blood.

<Subject and Method>

The subject is as follows. (1) 120 Samples of blood serum (early colon cancer 40, benign colon disorder 30, healthy state 50, the age was $63.2 \pm 12.8$ (mean $\pm$ SD), $61.8 \pm 14.1$, and $60.8 \pm 12.1$, respectively, same numbers of male and female), (2) 237 cases of cancer patient (breast cancer 58, stomach cancer 38, colon and rectum cancer 29, lung cancer 20, uterus cancer 18, ovary cancer 15, nasopharyngeal cancer 7, spleen cancer 5, live cancer 4, tongue cancer 1, and others 29), 100 cases of benign disorder, and 50 cases of healthy individual (age of from 22 to 81, 46.4 $\pm$ 13.5, male/female ratio of 2 : 3).

$\alpha$1-Globulin fraction and $\alpha$2-globulin fraction were measured by a blood serum protein fraction method based on cellulose acetate electrophoresis and the blood serum TPA was measured by using RIA two-antibody method. $\alpha$1-Globulin fraction, $\alpha$2-globulin fraction, and TPA were simultaneously measured, product of $\alpha$1 $\times$ $\alpha$2 and product of TPA $\times$ $\alpha$1 were calculated, and the product distribution, positive percentage, and mean $\pm$ SD were obtained for each case. Furthermore, for the subject (2), the cancer cases were classified for each cancer progress level (i.e., stages of from I to IV), and the positive percentage and mean $\pm$ SD were compared at each stage.

<Results of Measurement>

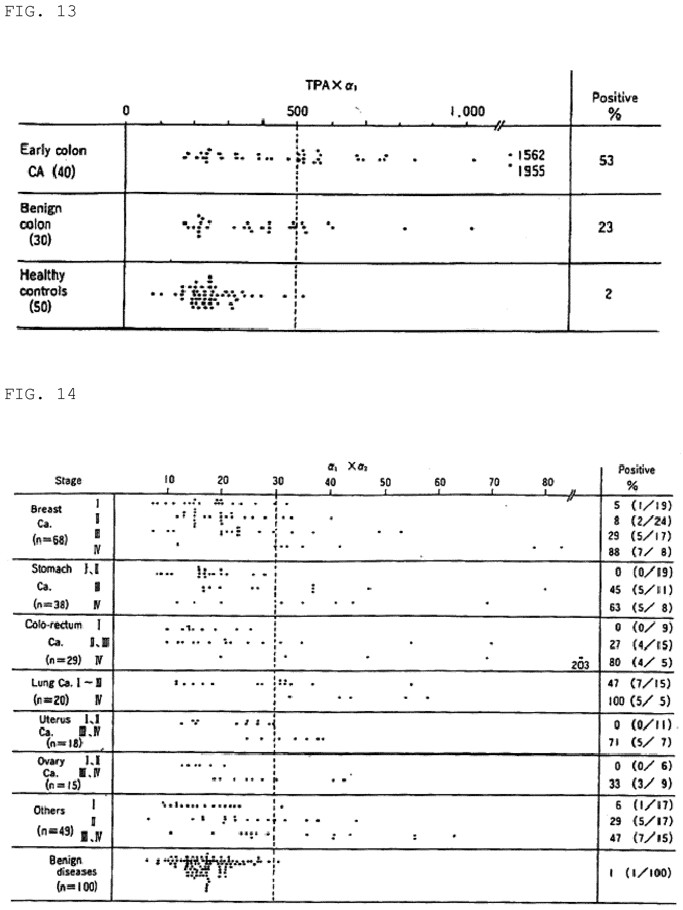

Fig. 12 is a distribution diagram illustrating the distribution state of a product value of blood serum $\alpha$1-globulin $\times$ $\alpha$2-globulin in early colon cancer when the classification is made according to the data collection method used for classifying the clinical cancer stage of Example 2, in which the upper column represents the early colon cancer (40 cases), the middle column represents benign tumor (30 cases), and the bottom column represents a healthy subject (50 cases). Fig. 13 is a distribution diagram illustrating the distribution state of a product value of blood serum TPA $\times$ $\alpha$1-globulin in early colon cancer when the classification is made according to the data collection method used for classifying the clinical cancer stage of Example 2, in which the upper column represents the early colon cancer (40 cases), the middle column represents benign tumor (30 cases), and the bottom column represents a healthy subject (50 cases).

Examination was made regarding the distribution of a product of $\alpha$1 $\times$ $\alpha$2 and a product of TPA $\times$ $\alpha$1 in early colon cancer. Herein, for mean + SD of a healthy subject case, $\alpha$1 $\times$ $\alpha$2 is 30.0, TPA $\times$ $\alpha$1 is 411.8, and the cut off value was set at 30 and 500, respectively. Mean $\pm$ SD of $\alpha$1-globulin $\times$ $\alpha$2-globulin and positive percentage for each disorder are as follows:

early colon cancer: 34.8 ($\pm$16.7), (48%),
benign colon disorder: 27.5 ($\pm$9.8), (20%),
healthy subject: 20.9 ($\pm$4.5), (2%),

in which a significant difference was recognized for each of those 3 cases.

For TPA $\times$ $\alpha$1-globulin, the values are as follows:

early colon cancer: 527.8 ($\pm$353.6), (53%),
benign colon disorder: 393.3 ($\pm$195), (23%),
healthy subject: 251.5 ($\pm$80.2), (2%),

in which a significant difference was recognized for each of those 3 cases.

Next, when combination assay is carried out for $\alpha$1-globulin $\times$ $\alpha$2-globulin and TPA $\times$ $\alpha$1-globulin, as shown in Table 2, those exhibiting a positive response for any one of them were 68% in early colon cancer, and the positive percentage was higher than a case of single presence. Cases showing positive response for both and cases showing negative response for both were all 32.5% (13/40), and they both tend to increase. The specificity found from the healthy subject group was as high as 96%, but it was slightly lower, i.e., 67%, in the benign group.

[Table 2]

Diagnosis ability of product of blood serum ($\alpha_1 \times \alpha_2$ and product of TPA $\times \alpha_1$ in early colon cancer (Mayo Clinic-NCI sample)

Cut off value was set as follows - $\alpha_1$: 3.3 (%), $\alpha_2$: 10.0 (%), TPA: 125 (U/l), $\alpha_1 \times (\alpha_2$: 30, TPA $\times \alpha_1$: 500.

| Marker | Sensitivity % | Specificity % | |
|---|---|---|---|
| | (n=40) | Benign (n=30) | Healthy (n=50) |
| $\alpha_1$ | 50 | 80 | 98 |
| $\alpha_2$ | 40 | 77 | 100 |
| TPA | 45 | 47 | 92 |
| $\alpha_1 \times \alpha_2$ | 48 | 80 | 98 |
| TPA $\times \alpha_1$ | 53 | 77 | 98 |
| $\alpha_1 \times \alpha_2$ and/or TPA $\times \alpha_1$ | 68 | 67 | 96 |

<Results from various cancer cases>

Fig. 14 is a distribution diagram examined against a product value of $\alpha$1-globulin $\times \alpha$2-globulin in various cancer cases.

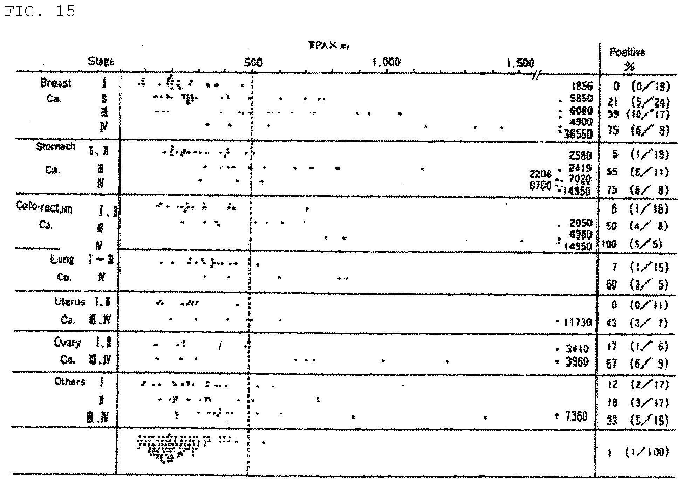

Fig. 15 is a distribution diagram examined against a product value of TPA $\times \alpha$1-globulin in various cancer cases.

Distribution of a product value of $\alpha$1-globulin $\times \alpha$2-globulin and a product value of TPA $\times \alpha$1-globulin in various cancer cases was examined for the samples. The positive percentage has increased for both in accordance with a progress of cancer regardless of cancer site. In addition, there was a relationship between the cancer progress rate and mean $\pm$ SD, and positive percentage (Table 3).

As for the $\alpha$1-globulin $\times \alpha$2-globulin, it is as follows:

stage I: 17.3 ($\pm$6.1),
stage II: 22.5 ($\pm$10.2),
stage III: 28.8 ($\pm$12.6),
stage IV: 44.3 ($\pm$32.9),

in which the positive percentage has increased to 6, 17, and 38.79%, respectively. Mean $\pm$ SD was 17.9 $\pm$ 4.6 and 17.7 $\pm$ 3.0 for the benign disorder and healthy subject, respectively, and false positive percentage was 1 and 0%. Although a significant difference was not recognized therebetween, a significant difference was recognized when compared to the cancer group (excluding stage I).

As for TPA $\times \alpha$1-globulin, it is as follows:

stage I: 308.5 ($\pm$403.2),
stage II: 356.6 ($\pm$236.5),
stage III: 723.5 ($\pm$1012.4),
stage IV: 3132.2 ($\pm$5624.2)

in which the positive percentage has increased to 6, 14, and 44.76%, respectively. Mean$\pm$SD was 223.6$\pm$90.0 and 205.9 ($\pm$40.7) for the benign disorder and healthy subject, respectively, and false positive percentage was 1 and 0%. Although a significant difference was not recognized therebetween, a significant difference was recognized when compared to the cancer group.

[Table 3]

Positive percentage of product value of blood serum $\alpha_1 \times \alpha_2$ and product value of TPA $\times \alpha_1$ for each progress stage of various cancers

Cut off values are shown in Table 1.

| Marker | Positive % | | | | |
|---|---|---|---|---|---|
| | Malignant (Stage) | | | | Benign |
| | I (n=66) | II (n=72) | III (n=66) | IV (n=33) | (n=100) |
| $\alpha_1$ | 11 | 15 | 48 | 85 | 2 |
| $\alpha_2$ | 5 | 10 | 29 | 27 | 1 |
| TPA | 24 | 30 | 64 | 76 | 13 |
| $\alpha_1 \times \alpha_2$ | 6 | 17 | 38 | 79 | 1 |
| TPA $\times \alpha_1$ | 6 | 14 | 44 | 76 | 1 |
| $\alpha_1 \times \alpha_2$ and/or TPA $\times \alpha_1$ | 11 | 26 | 58 | 88 | 2 |

Next, when combination assay is carried out for $\alpha$1-globulin $\times \alpha$2-globulin and TPA $\times \alpha$1-globulin, those exhibiting a positive response for any one of them showed an increase as follows in accordance with a progress of cancer;

I (stage): 11 (%),
II (stage): 26,
III (stage): 58,
IV (stage): 88.

Furthermore, when it is examined for each stage, the positive percentage has further increased compared to a case in which each is present singly. The value was 2 and 0% for the benign and healthy subject, respectively, and a significant difference was recognized when compared to the cancer group including early cancer.

Furthermore, the mean+2SD of the healthy subject group of this facility was as follows - $\alpha$1 $\times \alpha$2: 23.8, TPA $\times \alpha$1: 287.3.

It is shown that both the mean value and positive percentage of the product of $\alpha$1-globulin $\times \alpha$2-globulin and product TPA $\times \alpha$1-globulin increase in various cancer cases, and thus their usefulness as a tumor marker is demonstrated. In particular, the positive percentage is further increased in combination assay of both of them, and as it is useful for discriminating early cancer from benign case, an application to screening is expected.

<Setting of discriminant function>

1. Setting of discriminant function

Next, by using the data collection method used for classifying cancer in Example 2, 100 cases that are morphologically diagnosed as cancer and 100 cases not diagnosed as cancer were randomly selected, and the test was carried by having them as a patient group and a normal group, respectively. They are taken as sample A.

Analysis I: Data of sample A were directly analyzed.

Analysis II: Analysis was made after selecting 50 cases from each of a patient group in which liver disorder boundary values, and particularly abnormal values are excluded from the data of sample A, and also a normal group.

Analysis III: Each measured value from sample A was converted in terms of a distance from the normal range (Alb $\geq$ 58.0, al $\leq$ 3.0, $\alpha$2 $\leq$ 9.0) (i.e., distance from cut off value) and the resulting data (i.e., (Alb)'-(al)' and ($\alpha$2)', respectively) were analyzed. In case of Alb, for example, values higher than 58.0, all 0.57 are converted to 1.0 while 56.0 is converted to 2.0.

Furthermore, for the analysis, by using a program for obtaining each coefficient of a discriminant equation, value distribution of each, correlation or the like according to incorporation of data using a computer, the discriminant function was set.

<Determination of obtained discriminant function>

Separate from the above sample A, 100 cases were randomly selected from each of a patient group and a normal group, and employed as sample B. A discriminant function was determined for each.

<Clinical application of obtained discriminant function>

For test samples which have been additionally supplied (15 healthy cases, 20 early lung cancer cases, and 20 early colon cancer cases excluding benign tumor), cancer diagnosis was carried out based on the discriminant function which has been obtained by setting a discriminant function as described above.

<Formula for calculation>

As a calculation formula that is used for the data collection method used for classifying cancer in Example 2, it is considered that the following mathematical formula represented by Mathematical Formula 1 is useful.
In case of Z ≥ 0 according to the mathematical formula of Mathematical Formula 1, it is determined to be a normal healthy person (normal), while it is determined to be cancer in case of Z < 0.

[Mathematical Formula 1]

$$Z = 1.636 + 1.03\sqrt{(Alb)} - 2.73\sqrt{(\alpha 1)} - 0.805\sqrt{(\alpha 2)}$$

Sensitivity (hereinbelow, abbreviated as ser) to find a cancer to be cancer, specificity (hereinbelow, abbreviated as spe) to find a normal case to be normal, and total correct diagnosis rate of both are as follows when the mathematical formula of Mathematical Formula 1 is determined in sample A: sen 64%, spe 91%, and correct diagnosis rate 77.5%. Furthermore, in sample B, they were as follows: sen 75%, spe 92%, and correct diagnosis rate 83.5%. In separate sample other than those, they were as follows: sen 97.6%, spe 26.7%, and correct diagnosis rate 78%.
According to the data collection method used for classifying cancer in Example 2, a favorable correct diagnosis rate was obtained by using multivariate analysis of blood serum protein fraction. In this regard, it is believed that, although an abnormality appears in each fraction of different individuals according to growth of cancer, overall determination of them can be achieved by the method.

2) There has been a report of prior art literature that, when lung cancer is examined for a human physical examination subject using albumin and α2-globulin, discrimination rate of 55.2% can be obtained. It is found that the correct diagnosis rate can be further enhanced by adding α1-globulin thereto. Although albumin, α1-globulin, and α2-globulin were used for the classification method of Example 2, β-globulin fraction is less effective for cancer discrimination while having the same effect other than that.

<Biochemical biopsy of protein fragment>

Biochemical biopsy was carried out for a protein fragment. Namely, after collecting part of tissues or organs of a living body as a protein fragment, diagnosis of a disease was carried out. This biochemical biopsy can be carried out for a patient who is suspected to have cancer as malignant tumor, and it is the same as the pathological diagnosis which uses tissues like skin, stomach, intestine, liver, lung, and kidney.
According to electrophoresis, the protein fragment is electrophoresed to five basic bands. Among them, albumin, α1-globulin fraction, and α2-globulin fraction are deeply related to cancer growth. The first band corresponds to albumin, which is characteristically produced in a liver, and by regulating blood osmotic pressure, it helps the transport of materials and preservation of proteins. In case of liver disease, kidney disease, or systemic disease, reduced albumin is found.
The second band corresponds to α1-globulin fraction, in which α1-antitrypsin is present at 70 to 90%. It includes α1-acidic glycoprotein, α1-lipotprotein, prothrombin, transcortin, thyroxine, and binding globulin.
An increase in α1 fraction is yielded as an acute response to inflammation, cancer, stress, or hemorrhagic abnormality.
α1-Acidic glycoprotein is reduced in hepatitis, kidney disorder, or cachexia.
α1-Antitrypsin is a representative protease inhibitor for a proteinase. α1 Embryonic protein occurs in this fraction.
In α2-Globulin fraction, haptoglobin, α2-microglobulin, α2-HS glycoprotein, ceruloplasmin, erythropoetin, and choline esterase are included.
Haptoglobin works as it is bound to hemoglobin.
Increased haptoglobin is shown at early stage of cancer. α2-Microglobulin is present to be front-inclined in α2 band. α2-HS glycoprotein has a potential of a non-specific opsonin.

As a final item for confirmation, explanations are given for the characteristics of examples that are selected from the aforementioned embodiments.

<Data collection method A to be used for classifying cancer life>

Data collection method A to be used for classifying cancer life as one embodiment of the present invention is a data collection method to be used for classifying cancer life to discover development of minute cancer occurring in a human body and to perform data analysis of risk of tumor and risk of cancer in two divided stages of a preclinical cancer stage and a clinical cancer stage characterized in that:

for the data used for analysis of a preclinical cancer stage,
in order to collect data of an occurrence and a ratio of ALP I and activity value of ALP II and ALP III from patterns of the ALP I to the ALP IV as the ALP isozyme, examine the ratio of the numerical data, and perform data analysis for proliferation activity of cancer cells in view of APA calculated from ALP isozyme angle showing sharpness of the ALP II and the ALP III, and
additionally, for avoiding a cause contributed by an inflammatory disease, to perform an analysis while subtracting numerical data of a related part also occurring in the inflammatory disease from each numerical data obtained by measuring both the C reactive protein value (C inflammatory protein value, CRP value) and sialic acid value so as to perform data analysis for the existence and proliferation status of occurring minute cancer, and
for the data used for analysis of a clinical cancer stage,
to collect data from a changed state like a decrease in albumin fraction and an increase in $\alpha$1-globulin fraction, $\alpha$2-globulin fraction, and $\gamma$-globulin fraction that are shown in a protein fraction image and perform an analysis for each data, and
additionally, for avoiding a cause contributed by an inflammatory disease, during the data analysis of protein fraction image, to perform an analysis while subtracting numerical data of a related part also occurring in the inflammatory disease from each numerical data obtained by measuring both C reactive protein value (C inflammatory protein value, CRP value) and sialic acid value so as to perform data analysis for carrying out evaluation of the risk of tumor and the risk of cancer at several steps,
blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, and the data relating to the mobility, chromosome shape, and density of each isozyme are collected by matching a protein fraction image against the ALP isozyme.

<Data collection method B to be used for classifying cancer life>

Data collection method B to be used for classifying cancer life as one embodiment of the present invention is a data collection method to be used for classifying cancer life to discover development of minute cancer occurring in a human body and to perform data analysis of risk of tumor and risk of cancer in two divided stages of a preclinical cancer stage and a clinical cancer stage characterized in that:

for the data used for analysis of a preclinical cancer stage,
in order to collect data of an occurrence and a ratio of ALP I and activity value of ALP II and ALP III from patterns of the ALP I to the ALP IV as the ALP isozyme, examine the ratio of the numerical data, and perform data analysis for proliferation activity of cancer cells in view of APA calculated from ALP isozyme angle showing sharpness of the ALP II and the ALP III, and
additionally, for avoiding a cause contributed by an inflammatory disease, to perform an analysis while subtracting numerical data of a related part also occurring in the inflammatory disease from each numerical data obtained by measuring both C reactive protein value (C inflammatory protein value, CRP value) and sialic acid value so as to perform data analysis for the existence and proliferation status of occurring minute cancer, and
for the data used for analysis of a clinical cancer stage,
to perform data analysis such that the risk of cancer has four stages as follows for a cancer of 1 gram or more at a clinical cancer stage:

first stage in which albumin fraction is 65% or more, $\alpha$1-globulin fraction is less than 2.5%, and $\gamma$-globulin fraction is less than 16%,
second stage in which albumin fraction is 60% or more but less than 65%, $\alpha$1-globulin fraction is 2.5% or more but less than 3.0%, and $\gamma$-globulin fraction is 16% or more but less than 20%,
third stage in which albumin fraction is 55% or more but less than 60%, $\alpha$1-globulin fraction is 3.0% or more but less than 4.0%, and γ-globulin fraction is 20% or more but less than 23%, and

fourth stage in which albumin fraction is less than 55%, α1-globulin fraction is 4.0% or more, and γ-globulin fraction is 23% or more, and

at the same time, for avoiding a cause contributed by an inflammatory disease, during the data analysis of protein fraction image, to perform an analysis while subtracting numerical data of a related part also occurring in the inflammatory disease from each numerical data obtained by measuring both C reactive protein value (C inflammatory protein value, CRP value) and sialic acid value so as to perform data analysis for carrying out evaluation of the risk of tumor and the risk of cancer at several steps,

blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, and the data relating to the mobility, chromosome shape, and density of each isozyme are collected by matching the protein fraction image against the ALP isozyme.

<Data collection method C to be used for classifying cancer life>

Data collection method C to be used for classifying cancer life as one embodiment of the present invention is characterized in that, regarding data collection method A to be used for classifying cancer life or data collection method B to be used for classifying cancer life,

in order to use for the analysis of a preclinical cancer stage, for the data related to APA obtained from the ALP isozyme, an angle formed between a tangential line appearing in the positive electrode side of the ALP II and a line extending between a peak point of the ALP II and a peak point of the ALP III is set as $\theta_1°$, and

the distance from the cross point of those two tangential lines to a peak point of the ALP III is set as $\omega1$ cm, APA is expressed as follows : APA = $\omega1/\theta_1$, and when there is an occurrence of the ALP IV, the distance from the cross point with the tangential line of the ALP II to a peak point of the ALP III to the ALP IV is set as $\omega2$ cm, and APA is expressed as follows: APA = $\omega2/\theta_2$.

<Data collection method D to be used for classifying cancer life>

Data collection method D to be used for classifying cancer life as one embodiment of the present invention is characterized in that, regarding data collection method A to be used for classifying cancer life, data collection method B to be used for classifying cancer life, or data collection method C to be used for classifying cancer life,

for the data used for analysis of a preclinical cancer stage,

the blood serum is subjected to a heat treatment for 10 minutes at 56°C, data analysis is carried out for reconstituted patterns of each ALP isozyme including the ALP I to the ALP IV, and the ALP II, the ALP III, and the ALP IV are accurately identified.

<Data collection method E to be used for classifying cancer life>

Data collection method E to be used for classifying cancer life as one embodiment of the present invention is characterized in that, regarding data collection method D to be used for classifying cancer life,

for the data used for analysis of a preclinical cancer stage, data analysis is carried out for proliferation activity of cancer cells based on a change in each pattern of the ALP isozyme,

at the same time, data analysis is carried out for tumor growth with 5 stages in which tumor growth level is as follows based on tumor marker: stage I as an ideal state without having even a minute cancer, stage II as a precancer state at microgram level, stage III as precancer state at milligram level, stage IV as a preclinical cancer state, and stage V as a state assumed to have a existence of cancer of 1 g or more, and

data analysis is carried out in terms of the existence and proliferation status of minute cancer by employing in combination data analysis of the tumor marker over time.

<Data collection method F to be used for classifying cancer life>

Data collection method F to be used for classifying cancer life as one embodiment of the present invention is characterized in that, regarding data collection method A to be used for classifying cancer life or data collection method B to be used for classifying cancer life,

for the data used for analysis of a clinical cancer stage, an analysis is performed by subtracting α1-globulin fraction of a portion also occurring in an inflammatory disease from each numerical data of the measured C reactive protein value (C inflammatory protein value, CRP value) and measured sialic acid value.

# EP 3 425 404 A1

<Method G for classifying cancer life>

Method G for classifying cancer life as one embodiment of the present invention is a method for classifying a preclinical cancer stage in which blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, the mobility, chromosome shape, density, and the like of each isozyme are determined by matching the protein fraction image against the ALP isozyme, development of a minute cancer that is occurring is discovered, and also cancer life is classified by evaluating the risk of tumor and risk of cancer characterized in that:

an occurrence and a ratio of ALP I and activity value of ALP II and ALP III from patterns of the ALP I to the ALP IV as an ALP isozyme are examined, and proliferation activity of cancer cells is analyzed overall in view of APA values that are calculated from ALP isozyme angle showing sharpness of the ALP II and the ALP III, and

additionally, for avoiding a cause contributed by an inflammatory disease, determination is made by measuring both C reactive protein value (C inflammatory protein value, CRP value) and sialic acid value and subtracting the corresponding measured part to evaluate the existence and proliferation status of minute cancer that is occurring.

<Method H for classifying cancer life>

Method H for classifying cancer life as one embodiment of the present invention is characterized in that, regarding method G for classifying cancer life,

for the APA obtained from the ALP isozyme,

an angle formed between a tangential line appearing in the positive electrode side of the ALP II and a line extending between a peak point of the ALP II and a peak point of the ALP III is set as $\theta_1°$, and

the distance from the cross point of those two tangential lines to a peak point of the ALP III is set as $\omega 1$ cm, APA is expressed as follows : APA = $\omega 1/\theta_1$, and when there is an occurrence of the ALP IV, the distance from the cross point with the tangential line of the ALP II to a peak point of the ALP III to the ALP IV is set as $\omega 2$ cm, and APA is expressed as follows: APA = $\omega 2/\theta_2$.

<Method I for classifying cancer life>

Method I for classifying cancer life as one embodiment of the present invention is characterized in that, regarding method G for classifying cancer life or method H for classifying cancer life,

the blood serum is subjected to a heat treatment for 10 minutes at 56°C, data analysis is carried out for reconstituted patterns of each ALP isozyme including the ALP I to the ALP IV, and the ALP II, the ALP III, and the ALP IV are accurately identified.

<Method J for classifying cancer life>

Method J for classifying cancer life as one embodiment of the present invention is characterized in that, regarding method I for classifying cancer life,

proliferation activity of cancer cells is analyzed based on a change in each pattern of the ALP isozyme,

at the same time, data analysis is carried out for tumor growth with 5 stages in which tumor growth level is as follows based on tumor marker: stage I as an ideal state without having even a minute cancer, stage II as a precancer state at microgram level, stage III as precancer state at milligram level, stage IV as a preclinical cancer state, and stage V as a state assumed to have a existence of cancer of 1 g or more, and

the existence and proliferation status of minute cancer are determined by employing in combination data analysis of the tumor marker over time.

<Method K for classifying cancer life>

Method K for classifying cancer life as one embodiment of the present invention is a method for classifying a clinical cancer stage in which blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, the mobility, chromosome shape, density, and the like of each isozyme are determined by matching the protein fraction image against the ALP isozyme, development of a minute cancer that is occurring is discovered, and also cancer life is classified by evaluating the risk of tumor and risk of cancer characterized in that:

a changed state like a decrease in albumin fraction and an increase in $\alpha$1-globulin fraction, $\alpha$2-globulin fraction, and $\gamma$-globulin fraction that are shown in the protein fraction image is observed, and

for avoiding a cause contributed by an inflammatory disease, during evaluation of protein fraction image, determination is made by measuring both C reactive protein value (C inflammatory protein value, CRP value) and sialic acid value and subtracting the corresponding measured part to classify the risk of tumor and the risk of cancer into several stages.

<Method L for classifying cancer life>

Method L for classifying cancer life as one embodiment of the present invention is a method for classifying a clinical cancer stage in which blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, the mobility, chromosome shape, density, and the like of each isozyme are determined by matching the protein fraction image against the ALP isozyme, development of a minute cancer that is occurring is discovered, and also cancer life is classified by evaluating the risk of tumor and risk of cancer characterized in that:

with regard to cancer of 1 gram or more at a clinical cancer stage, when

first stage is a stage in which albumin fraction is 65% or more, $\alpha$1-globulin fraction is less than 2.5%, and $\gamma$-globulin fraction is less than 16%,

second stage is a stage in which albumin fraction is 60% or more but less than 65%, $\alpha$1-globulin fraction is 2.5% or more but less than 3.0%, and $\gamma$-globulin fraction is 16% or more but less than 20%,

third stage is a stage in which albumin fraction is 55% or more but less than 60%, $\alpha$1-globulin fraction is 3.0% or more but less than 4.0%, and $\gamma$-globulin fraction is 20% or more but less than 23%, and

fourth stage is a stage in which albumin fraction is less than 55%, $\alpha$1-globulin fraction is 4.0% or more, and $\gamma$-globulin fraction is 23% or more,

risk of cancer is classified into the four stages, and

at the same time, for avoiding a cause contributed by an inflammatory disease, during evaluation of protein fraction image, determination is made by measuring both C reactive protein value (C inflammatory protein value, CRP value) and sialic acid value and subtracting the corresponding measured part to evaluate the risk of tumor and the risk of cancer.

<Method M for classifying cancer life>

Method M for classifying cancer life as one embodiment of the present invention is characterized in that, regarding method G for classifying cancer life, method K for classifying cancer life, or method L for classifying cancer life,

depending on the height of the measured C reactive protein value (C inflammatory protein value, CRP value), the determination is made after subtracting the related part from $\alpha$1-globulin fraction.

Explanations are given for the effect of the above embodiments A to M.

According to the data collection method used for classifying a clinical cancer stage of the present invention, as a biochemical biopsy sample is added to a protein fraction, risk after clinical cancer, i.e., cancer of 1 gram or more, can be accurately classified. When the risk classification is made according to the method of the present invention, data allowing almost accurate numerical analysis of cancer prevention, recurrence prevention, therapeutic effect of cancer inhibiting agent, and progress state of cancer can be collected.

It is possible to determine clearly whether or not health food is effective for an individual, diet is proper, or cancer therapy is appropriate. If TMCA test is carried out before and after cancer operation, it is possible to identify early stage cancer or progressed cancer without having an open surgery. Of course, when an operation is carried out and comparison is made with the test result of TMCA before and after the operation, it is also possible to collect data that can be used for simple determination of the existence of remaining cancer or success or failure of the operation.

The data collection method used for classifying the life of cancer of the present invention corresponds to a health barometer, and it allows sure prevention of many misdiagnoses like determination made based on insufficient image diagnosis. Herein, TMCA test is a method for overall determination using tumor marker as described below. It allows collection of data enabling overall determination of toxicity in an internal environment of a living body, i.e., contamination level of an internal environment. It is a method for collecting data used for overall classification of risk based on determination of toxicity generated from cancer and so-called "tumor marker".

Furthermore, the change in protein fraction image is a phenomenon also occurring in an inflammatory disease. Thus, it is necessary to avoid the part contributed by the inflammatory disease. As such, both the C reactive protein value (C inflammatory protein value, CRP value) and a sialic acid value need to be measured. By carrying out the measurement in this way, more accurate classification of cancer can be achieved.

The data collection method used for classifying the life of cancer of the present invention can be carried out by general cancer classification. Although the standards may be slightly different in sarcoma or the like, it is possible to apply the data collection method used for classifying the life of cancer of the present invention.

It is possible that, depending on the height of the measured sialic acid value, the determination is made after subtracting the part also occurring in an inflammatory disease from $\alpha$1-globulin fraction so that the cause is determined to be cancer instead of an inflammatory disease by the evaluation.

According to the method for classifying a preclinical cancer stage of the present invention, since there is close relationship between cancer cell proliferation and APA, by analyzing the ALP isozyme pattern, minute cancer with cell number of $10^4$ to $10^9$ can be detected. Frequent appearance of the ALP I and also an increase in the ALP I in accordance with a progress of cancer can be determined. The ALP II is referred to as systemic ALP, and it is recognized to be involved with the ALP in every organ. It is known that, in case in which the total activity of the ALP has increased, an increase in the ALP III is shown in osteogenesis, liver cirrhosis, chronic kidney failure or the like. If the ALP is within a normal range, the ALP III increases in case of having new cell generation, for example, proliferation of minute cancer, regeneration of liver, and clinical cancer with slow progress.

Furthermore, according to the method for classifying a preclinical cancer stage of the present invention, as the progress of cancer accelerates in clinical cancer, the ALP III is changed to the ALP II according to an action of neuraminidase present in blood, thus yielding reduced ALP III. It is highly likely that the ALP III is a modification enzyme which has a dephosphrylating activity relating to proliferation of newly generated cells.

According to the method for classifying a preclinical cancer stage of the present invention, based on an analysis of reconstituted pattern of each ALP isozyme, the ALP II (ALP 2), the ALP III (ALP 3), and the ALP IV (ALP 4) can be identified with high precision and high rate. In particular, although the ALP IV is detected at high rate from cancer tissues, appearance frequency of the ALP IV in blood serum of a cancer patient is said to be 1 to 30%, and, due to the low activity, it is simply not observed or mistaken as the ALP III. According to the analysis of a reconstituted pattern, it is possible to detect the ALP IV at high rate.

According to the method for classifying a preclinical cancer stage of the present invention, when identification of the ALP isozyme pattern is not clear, by classifying the tumor stage based on a tumor marker and introducing a model for stage classification, accurate evaluation of the cancer development starting from minute cancer to clinical cancer can be achieved.

As described above, it is shown that the ALP isozyme I appears when there is an increase in total activity such as primary liver cancer, liver invasion, stasis liver, or fatty liver, or the like, the ALP II is referred to as hepatic the ALP and also recognized from pericardial water, an increase in the ALP III is shown in osteogenesis, liver cirrhosis, chronic kidney failure or the like when total the ALP activity increases, and there is a close relationship between cancer cell proliferation and the pattern of the ALP isozymes including the ALP I to the ALP IV, and thus, according to analysis of the pattern of those the ALP isozymes, minute cancer with cell number of $10^4$ to $10^9$ can be detected and risk of the minute cancer can be evaluated.

Furthermore, there is an excellent effect that, when identification of the ALP isozyme pattern is not clear, by classifying the tumor stage based on a tumor marker and introducing a model for stage classification, accurate evaluation of the cancer development starting from minute cancer to clinical cancer can be achieved.

According to the method for classifying a clinical cancer stage of the present invention, as a biochemical biopsy sample is added to a protein fraction, risk after clinical cancer, i.e., cancer of 1 gram or more, can be accurately classified. When the risk classification is made according to the method of the present invention, almost accurate numerical analysis of cancer prevention, recurrence prevention, therapeutic effect of cancer inhibiting agent, and progress state of cancer can be achieved.

It is possible to determine clearly whether or not health food is effective for an individual, diet is proper, or cancer therapy is appropriate. If TMCA test is carried out before and after cancer operation, it is possible to identify early stage cancer or progressed cancer without having an open surgery. Of course, when an operation is carried out and comparison is made with the test result of TMCA before and after the operation, it is also possible to perform simple determination of the existence of remaining cancer or success or failure of the operation.

The method for classifying the life of cancer of the present invention corresponds to a health barometer, and it allows sure prevention of many misdiagnoses like determination made based on insufficient image diagnosis. Herein, TMCA test is a method for overall determination using tumor marker as described below. It allows overall determination of toxicity in an internal environment of a living body, i.e., contamination level of an internal environment. It is a method for overall classification of risk based on determination of toxicity generated from cancer and so-called "tumor marker".

Furthermore, the change in protein fraction image is a phenomenon also occurring in an inflammatory disease. Thus, it is necessary to avoid the part contributed by the inflammatory disease. As such, both the C reactive protein value (C inflammatory protein value, CRP value) and a sialic acid value need to be measured. By carrying out the measurement in this way, more accurate classification of cancer can be achieved.

The method for classifying a clinical cancer stage of the present invention can be carried out by general cancer classification. Although the standards may be slightly different in sarcoma or the like, it is possible to apply the method for classifying a clinical cancer stage of the present invention.

Furthermore, regarding the method for classifying the life of cancer, it is possible that, depending on the height of the measured sialic acid value, the determination is made after subtracting it from $\alpha$1-globulin fraction so that the cause is determined to be cancer instead of an inflammatory disease by the evaluation.

Furthermore, as long as it is possible to contribute to prevention and treatment of cancers by not only finding early cancers in specific organs according to examining the existence of minute cancer in any part of a human body and simultaneously carrying out an ALP isozyme pattern analysis and a tumor marker analysis, and an analysis of blood serum protein fraction followed by overall evaluation, but also determining high-risk group for minute cancers present at any parts and early cancers of clinical cancer stages and classifying the stages of progressed cancers, and to suggest a scientific way to cope by precisely evaluating the progress of a treatment for a pre-existing disorder, it is evident that the present invention is not limited to the embodiments of the invention that are described above and various modifications can be made within a range not departing from the gist of the present invention.

Furthermore, as long as both the minute cancer at a preclinical cancer stage and cancer at a clinical cancer stage in the life of cancer can be detected and their risk can be appropriately determined and classified, it is evident that the present invention is not limited to the embodiments of the invention that are described above and various modifications can be made within a range not departing from the gist of the present invention.

Industrial Applicability

The present invention can be used for detecting all the data that are used for discovering minute cancer at a preclinical cancer stage and early cancer at a clinical cancer stage in the life of cancer, and appropriately determining and classifying the risk of cancers. The present invention can be utilized as a barometer for assessing the life of cancer. In other words, by employing the life of cancer, which is the biggest threat to humankind, as an unexpected means, utilization as a real barometer of health can be achieved.

**Claims**

1. A data collection method to be used for classifying cancer life to discover development of minute cancer occurring in a human body and to perform data analysis of risk of tumor and risk of cancer in two divided stages of a preclinical cancer stage and a clinical cancer stage, wherein,

   for the data used for analysis of a preclinical cancer stage,

   in order to collect data of an occurrence and a ratio of ALP I and activity value of ALP II and ALP III from patterns of the ALP I to the ALP IV as the ALP isozyme, examine the ratio of the numerical data, and perform data analysis for proliferation activity of cancer cells in view of APA calculated from ALP isozyme angle showing sharpness of the ALP II and the ALP III, and

   additionally, for avoiding a cause contributed by an inflammatory disease, to perform an analysis while subtracting numerical data of a related part also occurring in the inflammatory disease from each numerical data obtained by measuring both the C reactive protein value (C inflammatory protein value, CRP value) and sialic acid value so as to perform data analysis for the existence and proliferation status of occurring minute cancer, and

   for the data used for analysis of a clinical cancer stage,

   to collect data from a changed state like a decrease in albumin fraction and an increase in $\alpha$1-globulin fraction, $\alpha$2-globulin fraction, and $\gamma$-globulin fraction that are shown in a protein fraction image and perform an analysis for each data, and

   additionally, for avoiding a cause contributed by an inflammatory disease, during the data analysis of protein fraction image, to perform an analysis while subtracting numerical data of a related part also occurring in the inflammatory disease from each numerical data obtained by measuring both C reactive protein value (C inflammatory protein value, CRP value) and sialic acid value so as to perform data analysis for carrying out evaluation of the risk of tumor and the risk of cancer at several steps,

   blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, and the data relating to the mobility, chromosome shape, and density of each isozyme are collected by matching a protein fraction image against the ALP isozyme.

2. The data collection method to be used for classifying cancer life according to claim 1, wherein, in order to use for the analysis of a preclinical cancer stage, for the data related to APA obtained from the ALP isozyme, an angle formed between a tangential line appearing in the positive electrode side of the ALP II and a line extending between a peak point of the ALP II and a peak point of the ALP III is set as $\theta_1$°, and the distance from the cross point of those two tangential lines to a peak point of the ALP III is set as $\omega 1$ cm, APA is expressed as follows: APA = $\omega 1/\theta_1$, and when there is an occurrence of the ALP IV, the distance from the cross point with the tangential line of the ALP II to a peak point of the ALP III to the ALP IV is set as $\omega 2$ cm, and APA is expressed as follows: APA = $\omega 2/\theta_2$.

3. The data collection method to be used for classifying cancer life according to claim 1, wherein, for the data used for analysis of a preclinical cancer stage, the blood serum is subjected to a heat treatment for 10 minutes at 56°C, data analysis is carried out for reconstituted patterns of each ALP isozyme including the ALP I to the ALP IV, and the ALP II, the ALP III, and the ALP IV are accurately identified.

4. The data collection method to be used for classifying cancer life according to claim 2, wherein, for the data used for analysis of a preclinical cancer stage, the blood serum is subjected to a heat treatment for 10 minutes at 56°C, data analysis is carried out for reconstituted patterns of each ALP isozyme including the ALP I to the ALP IV, and the ALP II, the ALP III, and the ALP IV are accurately identified.

5. The data collection method to be used for classifying cancer life according to claim 1, wherein, for the data used for analysis of a clinical cancer stage, an analysis is performed by subtracting $\alpha 1$-globulin fraction of a related part also occurring in an inflammatory disease from each numerical data of the measured C reactive protein value (C inflammatory protein value, CRP value) and measured sialic acid value.

6. The data collection method to be used for classifying cancer life according to claim 3, wherein, for the data used for analysis of a preclinical cancer stage, data analysis is carried out for proliferation activity of cancer cells based on a change in each pattern of the ALP isozyme, at the same time, data analysis is carried out for tumor growth with 5 stages in which tumor growth level is as follows based on tumor marker: stage I as an ideal state without having even a minute cancer, stage II as a precancer state at microgram level, stage III as precancer state at milligram level, stage IV as a preclinical cancer state, and stage V as a state assumed to have a existence of cancer of 1 g or more, and data analysis is carried out in terms of the existence and proliferation status of minute cancer by employing in combination data analysis of the tumor marker over time.

7. The data collection method to be used for classifying cancer life according to claim 4, wherein, for the data used for analysis of a preclinical cancer stage, data analysis is carried out for proliferation activity of cancer cells based on a change in each pattern of the ALP isozyme, at the same time, data analysis is carried out for tumor growth with 5 stages in which tumor growth level is as follows based on tumor marker: stage I as an ideal state without having even a minute cancer, stage II as a precancer state at microgram level, stage III as precancer state at milligram level, stage IV as a preclinical cancer state, and stage V as a state assumed to have a existence of cancer of 1 g or more, and data analysis is carried out in terms of the existence and proliferation status of minute cancer by employing in combination data analysis of the tumor marker over time.

8. A data collection method to be used for classifying cancer life to discover development of minute cancer occurring in a human body and to perform data analysis of risk of tumor and risk of cancer in two divided stages of a preclinical cancer stage and a clinical cancer stage, wherein,
for the data used for analysis of a preclinical cancer stage,
in order to collect data of an occurrence and a ratio of ALP I and activity value of ALP II and ALP III from patterns of the ALP I to the ALP IV as the ALP isozyme, examine the ratio of the numerical data, and perform data analysis for proliferation activity of cancer cells in view of APA calculated from ALP isozyme angle showing sharpness of the ALP II and the ALP III, and
additionally, for avoiding a cause contributed by an inflammatory disease, to perform an analysis while subtracting numerical data of a related part also occurring in the inflammatory disease from each numerical data obtained by measuring both C reactive protein value (C inflammatory protein value, CRP value) and sialic acid value so as to perform data analysis for the existence and proliferation status of occurring minute cancer, and
for the data used for analysis of a clinical cancer stage,
to perform data analysis such that the risk of cancer has four stages as follows for a cancer of 1 gram or more at a clinical cancer stage:

first stage in which albumin fraction is 65% or more, $\alpha 1$-globulin fraction is less than 2.5%, and $\gamma$-globulin fraction is less than 16%,
second stage in which albumin fraction is 60% or more but less than 65%, $\alpha 1$-globulin fraction is 2.5% or more but less than 3.0%, and γ-globulin fraction is 16% or more but less than 20%,
third stage in which albumin fraction is 55% or more but less than 60%, α1-globulin fraction is 3.0% or more but less than 4.0%, and γ-globulin fraction is 20% or more but less than 23%, and
fourth stage in which albumin fraction is less than 55%, α1-globulin fraction is 4.0% or more, and γ-globulin fraction is 23% or more, and
at the same time, for avoiding a cause contributed by an inflammatory disease, during the data analysis of protein fraction image, to perform an analysis while subtracting numerical data of a related part also occurring in the inflammatory disease from each numerical data obtained by measuring both C reactive protein value (C inflammatory protein value, CRP value) and sialic acid value so as to perform data analysis for carrying out evaluation of the risk of tumor and the risk of cancer at several steps,
blood serum is applied on a support that has been impregnated in a buffer solution, the support is electrophoretically fractionated at a predetermined liquid temperature to isolate proteins in the blood serum, the ALP isozyme is detected by color-developing with an ALP isozyme staining solution, and the data relating to the mobility, chromosome shape, and density of each isozyme are collected by matching the protein fraction image against the ALP isozyme.

9. The data collection method to be used for classifying cancer life according to claim 8, wherein, in order to use for the analysis of a preclinical cancer stage, for the data related to APA obtained from the ALP isozyme, an angle formed between a tangential line appearing in the positive electrode side of the ALP II and a line extending between a peak point of the ALP II and a peak point of the ALP III is set as $\theta_1$°, and the distance from the cross point of those two tangential lines to a peak point of the ALP III is set as $\omega 1$ cm, APA is expressed as follows: APA = $\omega 1/\theta_1$, and when there is an occurrence of the ALP IV, the distance from the cross point with the tangential line of the ALP II to a peak point of the ALP III to the ALP IV is set as $\omega 2$ cm, and APA is expressed as follows: APA = $\omega 2/\theta_2$.

10. The data collection method to be used for classifying cancer life according to claim 8, wherein, for the data used for analysis of a preclinical cancer stage, the blood serum is subjected to a heat treatment for 10 minutes at 56°C, data analysis is carried out for reconstituted patterns of each ALP isozyme including the ALP I to the ALP IV, and the ALP II, the ALP III, and the ALP IV are accurately identified.

11. The data collection method to be used for classifying cancer life according to claim 9, wherein, for the data used for analysis of a preclinical cancer stage, the blood serum is subjected to a heat treatment for 10 minutes at 56°C, data analysis is carried out for reconstituted patterns of each ALP isozyme including the ALP I to the ALP IV, and the ALP II, the ALP III, and the ALP IV are accurately identified.

12. The data collection method to be used for classifying cancer life according to claim 8, wherein, for the data used for analysis of a clinical cancer stage, an analysis is performed by subtracting α1-globulin fraction of a related part also occurring in an inflammatory disease from each numerical data of the measured C reactive protein value (C inflammatory protein value, CRP value) and measured sialic acid value.

13. The data collection method to be used for classifying cancer life according to claim 10, wherein, for the data used for analysis of a preclinical cancer stage, data analysis is carried out for proliferation activity of cancer cells based on a change in each pattern of the ALP isozyme, at the same time, data analysis is carried out for tumor growth with 5 stages in which tumor growth level is as follows based on tumor marker: stage I as an ideal state without having even a minute cancer, stage II as a precancer state at microgram level, stage III as precancer state at milligram level, stage IV as a preclinical cancer state, and stage V as a state assumed to have a existence of cancer of 1 g or more, and data analysis is carried out in terms of the existence and proliferation status of minute cancer by employing in combination data analysis of the tumor marker over time.

14. The data collection method to be used for classifying cancer life according to claim 11, wherein, for the data used for analysis of a preclinical cancer stage, data analysis is carried out for proliferation activity of cancer cells based on a change in each pattern of the ALP isozyme, at the same time, data analysis is carried out for tumor growth with 5 stages in which tumor growth level is as follows based on tumor marker: stage I as an ideal state without having even a minute cancer, stage II as a precancer state at microgram level, stage III as precancer state at milligram level, stage IV as a preclinical cancer state, and stage V as a state assumed to have a existence of cancer of 1 g or more, and data analysis is carried out in terms of the existence and proliferation status of minute cancer by employing in combination data analysis of the tumor marker over time.

FIG. 1

| | | |
|---|---|---|
| PRECANCER STAGE | ◎ | NORMAL CELLS |
| (CANCERRATION) | ⬡ | CANCER CELLS |
| | | MINUTE CANCER<br>1mm (10⁶Cells) |
| PRECLINICAL<br>CANCER STAGE | | 7～8mm |
| CLINICAL<br>CANCER STAGE | ● | EARLY CANCER<br>10mm (10⁹Cells) |
| METASTASIS<br>METASTASIS | ● | PROGRESSED CANCER |

FIG. 2

```
                            ┌─────────────────┐
                            │     START       │
                            └─────────────────┘
                                    │
<FIRST STEP>                        ▼
EVALUATION OF        ┌──────────────────────────────┐
PRECLINICAL         │    EVALUATION BASED           │
CANCER STAGE        │    ON ALP ISOZYME             │
                    └──────────────────────────────┘
                                    │
                                    ▼
                    ┌──────────────────────────────┐
                    │  CLASSIFY INTO 5 TUMOR        │
                    │  STAGE                        │
                    └──────────────────────────────┘
                                    │
                                    ▼
       ┌────────────────────────────────────────────────────────┐
       │ PERFORM DETERMINATION WITH SUBTRACTION FROM α1-         │
       │ GLOBULIN FRACTION IN ACCORDANCE WITH MEASUREMENT        │
       │ VALUES OF CRP VALUE AND SIALIC ACID VALUR              │
       └────────────────────────────────────────────────────────┘
                                    │
                                    ▼
                          ◇ INFLAMATORY ◇        YES
                          ◇ DISORDER OR ◇ ──────────► BENIGN TUMOR,
                          ◇    NOT?     ◇            AND OTHERS
                                    │ NO
<SECOND STEP>                       ▼
EVALUATION OF        ┌──────────────────────────────┐
CLINICAL            │  EVALUATE PROTEIN FRAGMENT    │
CANCER STAGE        │  ANALYSIS BASED ON LEVEL      │
                    │  OF SPECIFIC TUMOR MARKER     │
                    └──────────────────────────────┘
                                    │
                                    ▼
                    ┌──────────────────────────────┐
                    │  CLASSIFY CANCER RISK         │
                    │  INTO 4 STAGES                │
                    └──────────────────────────────┘
                                    │
                                    ▼
       ┌────────────────────────────────────────────────────────┐
       │ PERFORM DETERMINATION WITH SUBTRACTION FROM α1-         │
       │ GLOBULIN FRACTION IN ACCORDANCE WITH MEASUREMENT        │
       │ VALUES OF CRP VALUE AND SIALIC ACID VALUR              │
       └────────────────────────────────────────────────────────┘
                                    │
                                    ▼
                          ◇ INFLAMATORY ◇        YES
                          ◇ DISORDER OR ◇ ──────────► BENIGN TUMOR,
                          ◇    NOT?     ◇            AND OTHERS
                                    │ NO
                                    ▼
                    ┌─────────────────┐      ┌─────────────────┐
                    │ COMPLETE CANCER │      │  TO OTHER TEST  │
                    │ CLASSIFICATIONR │      └─────────────────┘
                    └─────────────────┘
```

FIG. 3

(a)                                              (b)

II  III                              II  III IV

METHOD FOR MEASURING ALP ISOZYME ANGLE (AP-A)

(a)

WHEN ALP IV IS NOT RECOGNIZED,
ANGLE FORMED BETWEEN TANGENTIAL
LINE APPEARING IN POSITIVE
ELECTRODE SIDE OF ALP II AND LINE
EXTENDING BETWEEN PEAK POINT OF
ALP II AND PEAK POINT OF ALP III
IS SET AS $\theta_1$.
IF DISTANCE FROM THEIR CROSS POINT
TO PEAK POINT OF ALP III IS SET AS
$\alpha 1$ cm, AP-A IS EXPRESSED AS
FOLLOWS: AP-A = $\omega 1/\theta_1$.

(b)

WHEN EXISTENCE OF ALP IV IS
CONSIDERED, ANGLE FORMED
BETWEEN TANGENTIAL LINE OF ALP
II AND TANGENTIAL LINE OF ALP
IV IS SET AS $\theta'_2$, AND DISTANCE
FROM THEIR CROSS POINT TO PEAK
POINT OF ALP IV IS SET AS $\beta$
cm, AND AP-A IS EXPRESSED AS
FOLLOWS: A-PA = $\omega 2/\theta_2$.

FIG. 4

**(a)**

**(b)**

| DAYS POINTS | -15 | -9 | 48 | 99 |
|---|---|---|---|---|
| ALP$_I$(%) | 0 | 0 | 1 | 0 |
| ALP $_{II/III}$ | 0.6 | 0.7 | 1.8 | 1.3 |
| A P-A | 0.110 | 0.143 | 0.105 | 0.062 |

| MONTNS POINTS | 0 | 3 | 6 | 7 |
|---|---|---|---|---|
| ALP$_I$(%) | 1 | 2 | 17 | 20 |
| ALP $_{I/III}$ | 1.3 | 3.3 | 3.6 | 5.2 |
| A P-A | 0.161 | 0.222 | 0.276 | 0.381 |
| CEA(NG/ML) | 2.0 | 2.5 | 7.7 | 14.2 |

(a)

ANALYSIS OF BLOOD SERUM ALP ISOZYME FROM PATIENT WITH STAGE 1 RECTAL CANCER: FOR ALP ELECTROPHORESIS, KIT USING CELLULOSE ACETATE MEMBRANE (TITAN III) OF HELENA LABORATORIES AND DENSITOMETER WERE USED. NUMBERS GIVEN IN DRAWING INDICATE NUMBER OF DAYS, EITHER BEFORE OR AFTER OPERATION, WHEN DAY OF OPERATION IS SET AT ZERO.

(b)

ANALYSIS OF BLOOD SERUM ISOZYME FROM PATIENT HAVING ESOPHAGEAL CANCER WITH LIVER METASTASIS (56 YEAR OLD MALE): MONTH BY MONTH, WIDER LIVER METASTASIS AND LOWER ACTIVITY OF ALP III WERE SHOWN.

FIG. 5

(a)

| POINTS \ DAYS | -7(PRE) | 1 | 18 | 24 |
|---|---|---|---|---|
| ALP$_I$(%) | 8 | 0 | 5 | 0 |
| ALP II/III | 1.9 | 1.4 | 1.9 | 4.7 |
| AP-A | 0.207 | 0.150 | 0.164 | 0.294 |
| Δα-FT | 5.0 | 3.0 | 0 | 0 |
| ΔCEA | 0.3 | 0.6 | 0.1 | 0 |

(a)

ANALYSIS OF BLOOD SERUM ALP
ISOZYME FROM PATIENT WITH
EARLY BREAST CANCER: NUMBERS
GIVEN IN DRAWING INDICATE
NUMBER OF DAYS, EITHER BEFORE
OR AFTER OPERATION, WHEN DAY
OF OPERATION IS SET AT ZERO.

(b)

| POINTS \ DAYS | -138 | -11 | 0(OPE) | 8(POST) |
|---|---|---|---|---|
| ALP$_I$(%) | 0 | 0 | 0 | 1 |
| ALP II/III | 0.59 | 1.2 | 1.3 | 1.5 |
| AP-A | 0.114 | 0.159 | 0.121 | 0.100 |
| ΔCEA | 0.3 | 0 | 0.7 | 0 |
| ΔFERRITIN | 0 | 2 | 13 | 2 |

(b)

ANALYSIS OF BLOOD SERUM ALP
ISOZYME FROM PATIENT HAVING
RECURRENCE OF EARLY BREAST
CANCER: NUMBERS GIVEN IN DRAWING
INDICATE NUMBER OF DAYS, EITHER
BEFORE OR AFTER RE-OPERATION
CAUSED BY RECURRENCE, WHEN DAY
OF RE-OPERATION IS SET AT ZERO.

FIG. 6

EFFECT OF HEAT TREATMENT ON EACH BLOOD SERUM

(NUMBERS GIVEN IN DRAWING INDICATE TIME (MINUTES) FOR HEAT TREATMENT AT

56°C)

(a) BLOOD SERUM OF CANCER PATIENT IN WHICH ALP II IS PRESENT AS MAJOR
COMPONENT

(b) BLOOD SERUM OF 2-YEAR OLD CHILD IN WHICH ALP III IS PRESENT AS MAJOR
COMPONENT

(c) BLOOD SERUM OF WOMAN IMMEDIATELY AFTER DELIVERING BABY IN WHICH ALP
IV IS PRESENT AS MAJOR COMPONENT

(d) RECONSTITUTION TEST 1: COMBINATION OF ALP II AND ALP III

(e) RECONSTITUTION TEST 2: ALP IV WAS ADDED IN SMALL AMOUNT TO MIXTURE OF
ALP II AND ALP III (30% AND 70%, RESPECTIVELY)

(f) RECONSTITUTION TEST 3: COMBINATION OF ALP II AND ALP IV

FIG. 7

(a)

TUMOR MARKER INDUCTION IN
PATIENT WITH STAGE IV GALL
BLADDER CANCER (50 YEAR OLD
FEMALE): ACCORDING TO
INDUCTION BY VITAMIN A
(50000 IU) AND FAR INFRARED
RAY HYPERTHERMIA, FERRITIN
AND α-FP ARE RAPIDLY

(b)

TUMOR MARKER INDUCTION IN
MINUTE CANCER
ΔFERRITIN WAS CLASSIFIED INTO
3 TYPES OF INCREASING TYPE,
CONSTANT TYPE, AND DECREASING
TYPE

FIG. 8

(a)                    (b)

| HR AFTER HYPERTHER POINTS | 0 | 36 | 48 | 72 |
|---|---|---|---|---|
| ALP$_I$ (%) | 0 | 0 | 0 | 0 |
| ALP $_{II}/_{III}$ | 1.1 | 0.85 | 1.04 | 1.07 |
| A P-A | 0.124 | 0.138 | 0.110 | 0.116 |

| HR AFTER HYPERTHER POINTS | 0 | 6 | 36 | 72 |
|---|---|---|---|---|
| ALP$_I$ (%) | 0 | 0 | 0 | 0 |
| ALP $_{II}/_{III}$ | 1.4 | 1.1 | 1.2 | 1.26 |
| A P-A | 0.170 | 0.117 | 0.113 | 0.114 |

ALP ISOZYME ANALYSIS OF (a) ΔFERRITIN INCREASING
TYPE AND (b) ΔFERRITIN DECREASING TYPE

FIG. 9

FIG. 10

FRACTION No.

FIG. 11

PROTEIN FRACTION ANALYSIS CHART

FIG. 12

| | $\alpha_1$ $\times \alpha_2$ | Positive % |
| --- | --- | --- |
| | 10     30     50     70 | |
| Early colon CA (40) | | 48 |
| Benign colon (30) | | 20 |
| Healthy controls (50) | | 2 |

FIG. 13

FIG. 14

FIG. 15

| | Stage | TPAX α) 500 1,000 1,500 | | Positive % |
|---|---|---|---|---|
| Breast Ca. | I | 1856 | 0 | (0/19) |
| | II | 5850 | 21 | (5/24) |
| | III | 6080 | 59 | (10/17) |
| | | 4900 | | |
| | IV | 36550 | 75 | (6/8) |
| Stomach Ca. | I、II | 2580 | 5 | (1/19) |
| | III | 2208 · 2419 | 55 | (6/11) |
| | | 7020 | | |
| | IV | 6760 14950 | 75 | (6/8) |
| Colo-rectum Ca. | I、II | | 6 | (1/16) |
| | III | 2050 | 50 | (4/8) |
| | | 4980 | | |
| | IV | 14950 | 100 | (5/5) |
| Lung Ca. | I～III | | 7 | (1/15) |
| | IV | | 60 | (3/5) |
| Uterus Ca. | I、II | | 0 | (0/11) |
| | III、IV | 11730 | 43 | (3/7) |
| Ovary Ca. | I、II | 3410 | 17 | (1/6) |
| | III、IV | 3960 | 67 | (6/9) |
| Others | I | | 12 | (2/17) |
| | II | | 18 | (3/17) |
| | III、IV | 7360 | 33 | (5/15) |
| | | | 1 | (1/100) |

40

FIG. 16

FIG. 17

## INTERNATIONAL SEARCH REPORT

| International application No. |
|---|
| PCT/JP2017/007406 |

**A. CLASSIFICATION OF SUBJECT MATTER**
*G01N33/68*(2006.01)i, *G01N27/447*(2006.01)i, *C12Q1/42*(2006.01)n

According to International Patent Classification (IPC) or to both national classification and IPC

**B. FIELDS SEARCHED**

Minimum documentation searched (classification system followed by classification symbols)
G01N33/68, G01N27/447, C12Q1/42

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2002-181825 A  (Tsuneo KOBAYASHI), 26 June 2002 (26.06.2002), entire text; all drawings & US 2003/0108962 A1 entire text; all drawings | 1–14 |
| A | WO 2007/029300 A1  (Tsuneo KOBAYASHI), 15 March 2007 (15.03.2007), entire text; all drawings (Family: none) | 1–14 |
| A | JP 2001-289861 A  (Tsuneo KOBAYASHI), 19 October 2001 (19.10.2001), entire text; all drawings & WO 2003/036301 A1 entire text; all drawings | 1–14 |

☒  Further documents are listed in the continuation of Box C.   ☐   See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| 08 May 2017 (08.05.17) | 16 May 2017 (16.05.17) |

| Name and mailing address of the ISA/ <br> Japan Patent Office <br> 3-4-3,Kasumigaseki,Chiyoda-ku, <br> Tokyo 100-8915,Japan | Authorized officer <br><br> Telephone No. |
|---|---|

Form PCT/ISA/210 (second sheet) (January 2015)

**INTERNATIONAL SEARCH REPORT**

| International application No. |
| --- |
| PCT/JP2017/007406 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 62-100661 A  (Tsuneo KOBAYASHI),<br>11 May 1987 (11.05.1987),<br>entire text; all drawings<br>(Family: none) | 1-14 |
| A | WO 2012/011485 A1  (Taiho Pharmaceutical Co.,<br>Ltd.),<br>26 January 2012 (26.01.2012),<br>claims<br>& TW 201206427 A | 1-14 |
| A | JP 2012-517607 A  (Onconome, Inc.),<br>02 August 2012 (02.08.2012),<br>claims<br>& US 2012/0149022 A1<br>claims<br>& WO 2010/096154 A2    & EP 2398918 A2 | 1-14 |
| A | JP 4-244100 A  (Daiichi Radioisotope<br>Laboratories, Ltd.),<br>01 September 1992 (01.09.1992),<br>column 3, lines 5 to 8<br>(Family: none) | 1-14 |
| A | JP 2015-096508 A  (Bioмérieux),<br>21 May 2015 (21.05.2015),<br>paragraph [0031]<br>& US 2010/0136539 A1<br>paragraph [0048]<br>& WO 2009/019365 A2    & EP 2167971 A2 | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006302222 A